# EUROPEAN PATENT SPECIFICATION

(11) **EP 2 046 958 B1**
(45) Date of publication and mention of the grant of the patent: **23.03.2016**
(21) Application number: 07784751.5
(22) Date of filing: 03.08.2007
(51) Int. Cl.: C07K 14/47, A61K 38/00, C07K 14/485, C12Q 1/37

(54) **CLEAVAGE OF BIP BY SUBTILASE CYTOTOXIN**
SPALTUNG VON BIP DURCH SUBTILASE-ZYTOTOXIN
CLIVAGE DE BIP PAR UNE CYTOTOXINE SUBTILASE

(30) Priority: 04.08.2006 AU 2006904215
(43) Date of publication of application: 15.04.2009
(73) Proprietor: Adelaide Research And Innovation, Adelaide, SA 5000 (AU); SibTech, Inc., Newington, CT 06111 (US)
(72) Inventor: PATON, Adrienne Webster, Parkside, SA 5063 (AU); PATON, James Cleland, Parkside, SA 5063 (AU); BACKER, Marina V., West Simsbury, CT 06111 (US); BACKER, Joseph M., West Simsbury, CT 06111 (US)
(74) Representative: Nederlandsch Octrooibureau
(86) International application number: PCT/AU2007/001111
(87) International publication number: WO 2008/014574

(56) References cited:
- WO-A1-2005/061714
- US-A- 4 743 679
- US-A1- 2005 136 424
- JAMORA C ET AL: "Inhibition of tumor progression by suppression of stress protein GRP78/Bip induction in fibrosarcoma B/C10ME" PROCEEDINGS OF THE NATIONAL ACADEMY OF SCIENCES OF USA, NATIONAL ACADEMY OF SCIENCE, WASHINGTON, DC, US, vol. 93, no. 15, 23 July 1996 (1996-07-23) , pages 7690-7694, XP002960237 ISSN: 0027-8424
- LI JIANZE ET AL: "Stress induction of GRP78/BiP and its role in cancer" CURRENT MOLECULAR MEDICINE, BENTHAM SCIENCE PUBLISHERS, NL, vol. 6, no. 1, 1 February 2006 (2006-02-01), pages 45-54, XP009128660 ISSN: 1566-5240
- LASS A ET AL: "Decreased ER-associated degradation of alpha-TCR induced by Grp78 depletion with the SubAB cytotoxin" INTERNATIONAL JOURNAL OF BIOCHEMISTRY AND CELL BIOLOGY, EXETER, GB, vol. 40, no. 12, 1 January 2008 (2008-01-01), pages 2865-2879, XP025409763 ISSN: 1357-2725 [retrieved on 2008-06-20]
- PATON A.W. ET AL.: 'AB5 subtilase cytotoxin inactivates the endoplasmic reticulum chaperone BiP' NATURE vol. 443, no. 7111, 2006, pages 548 - 552, XP008102896
- PATON A.W. ET AL.: 'A New Family of Potent AB5 Cytotoxins Produced by Shiga Toxigenic Escherichia coli' THE JOURNAL OF EXPERIMENTAL MEDICINE vol. 200, no. 1, 2004, pages 35 - 46, XP008102899
- PATON A.W. AND PATON J.C.: 'Multiplex PCR for Direct Detection of Shiga Toxigenic Escherichia coli Strains Producing the Novel Subtilase Cytotoxin' JOURNAL OF CLINICAL MICROBIOLOGY vol. 43, no. 6, 2005, pages 2944 - 2947, XP008102900
- YAHIRO K. ET AL.: 'Identification and characterization of receptors for vacuolating activity of subtilase cytotoxin' MOLECULAR MICROBIOLOGY vol. 62, no. 2, 2006, pages 480 - 490, XP008102897
- MORINAGA N. ET AL.: 'Two Distinct Cytotoxic Activities of Subtilase Cytotoxin Produced by Shiga-Toxigenic Escherichia coli' INFECTION AND IMMUNITY vol. 75, no. 1, 2007, pages 488 - 496, XP008102901

### Sequence Listing

<110> University of Adelaide,and Subtech Corporation
<120> Cleavage of BiP by Subtilase Cytotoxin
<160> 21
<210> 1
   <211> 35
   <212> DNA
   <213> Artificial Sequence
<223> Primer
<400> 1
   cacaagccat gggaaacagc gatagcgaat gcccg 35
<210> 2
   <211> 33
   <212> DNA
   <213> Artificial Sequence
   <223> Primer
<400> 2
   actacccatg gagcgcagtt cccaccattt cag 33
<210> 3
   <211> 32
   <212> DNA
   <213> Artificial Sequence
   <223> Primer
<400> 3
   cacggatccg aaaaaccctg gtattttgat gc 32
<210> 4
   <211> 30
   <212> DNA
   <213> Artificial Sequence
   <223> Primer
<400> 4
   cacggatcct tacagttctt cactcttccc 30
<210> 5
   <211> 34
   <212> DNA
   <213> Artificial Sequence
   <223> Primer
<400> 5
   acaaacatca aggtctacca gatcacctgt atcc 34
<210> 6
   <211> 35
   <212> DNA
   <213> Artificial Sequence
   <223> Primer
<400> 6
   ggtgatctgg tagaccttga tgtttgtccc cttac 35
<210> 7
   <211> 10
   <212> PRT
   <213> Bovine
   <223> peptide fragment equivalent to amino Acids 417 - 426 of BiP
<400> 7
   Leu Asp Val Cys Pro Leu Thr Leu Gly Ile
   1               5                   10
<210> 8
   <211> 326
   <212> PRT
   <213> Escherichia coli
   <223> fragment of Subunit A of subtilase cytotoxin
<400> 8
<210> 9
   <211> 53
   <212> PRT
   <213> human
   <223> epidermal growth hormone
<400> 9
   Asn Ser Asp Ser Glu Cys Pro Leu Ser His Asp Gly Tyr Cys Leu
   1               5                   10                  15
   His Asp Gly Val Cys Met Tyr Ile Glu Ala Leu Asp Lys Tyr Ala
                   20                  25                  30
   Cys Asn Cys Val Val Gly Tyr Ile Gly Glu Arg Cys Gln Tyr Arg
                   35                  40                  45
   Asp Leu Lys Trp Trp Glu Leu Arg
                   50          53
<210> 10
   <211> 16
   <212> PRT
   <213> artificial sequence
   <223> cys tag
<400> 10
   Met Lys Glu Ser Cys Ala Lys Lys Phe Gln Arg Gln His Met Asp
   1               5                   10                  15
   Ser
   16
<210> 11
   <211> 17
   <212> PRT
   <213> artificial sequence
   <223> linker sequence
<400> 11
   Gly Gly Gly Gly Ser Gly Gly Gly Gly Ser Gly Gly Gly Gly Ser
   1               5                   10                  15
   Met Gly
       17
<210> 12
   <211> 7
   <212> PRT
   <213> artificial sequence
   <223> linker sequence
<400> 12
   Ser Met Ala Asp Ile Gly Ser
   1               5       7
<210> 13
   <211> 419
   <212> PRT
   <213> fusion protein
<223> of subA and human Egf wih cys tage consisting of SEQ ID NOS 7 to 12
<400> 13
<210> 14
   <211> 32
   <212> DNA
   <213> Artificial Sequence
   <223> Primer
<400> 14
   cacaagccat ggcacccatg gcagaaggag ga 32
<210> 15
   <211> 38
   <212> DNA
   <213> Artificial Sequence
   <223> Primer
<400> 15
   actacccatg gctcttgctc tatctttctt tggtctgc 38
<210> 16
   <211> 110
   <212> PRT
   <213> human
   <223> 110 amino acid fragment of vascular endothelial growth hormone 121
<400> 16
<210> 17
   <211> 464
   <212> PRT
   <213> fusion protein
   <223> of subA and human vegf with cys tag consisting of SEQ ID NOs 8,10,12,16 and 18
<400> 17
<210> 18
   <211> 5
   <212> PRT
   <213> artificial sequence
   <223> linker sequence
<400> 18
   Gly Gly Gly Gly Ser
   1 5
<210> 19
   <211> 353
   <212> PRT
   <213> fusion protein
   <223> of Cys tag and SubA consisting of SEQ ID NOs 10,20 and 8
<400> 19
   Met Lys Glu Ser Cys Ala Lys Lys Phe Gln Arg Gln His Met Asp
   1               5                   10                  15
   Ser Gly Gly Gly Gly Ser Met Ala Asp Ile Gly Ser Glu Lys Pro
                   20                  25                  30
   Trp Tyr Phe Asp Ala Ile Gly Leu Thr Glu Thr Thr Met Ser Leu
                   35                  40                  45
   Thr Asp Lys Asn Thr Pro Val Val Val Ser Val Val Asp Ser Gly
                   50                  55                  60
   Val Ala Phe Ile Gly Gly Leu Ser Asp Ser Glu Phe Ala Lys Phe
                   65                  70                  75
   Ser Phe Thr Gln Asp Gly Ser Pro Phe Pro Val Lys Lys Ser Glu
                   80                  85                  90
   Ala Leu Tyr Ile His Gly Thr Ala Met Ala Ser Leu Ile Ala Ser
                   95                  100                 105
   Arg Tyr Gly Ile Tyr Gly Val Tyr Pro His Ala Leu Ile Ser Ser
                   110                 115                 120
   Arg Arg Val Ile Pro Asp Gly Val Gln Asp Ser Trp Ile Arg Ala
                   125                 130                 135
   Ile Glu Ser Ile Met Ser Asn Val Phe Leu Ala Pro Gly Glu Glu
                   140                 145                 150
   Lys Ile Ile Asn Ile Ser Gly Gly Gln Lys Gly Val Ala Ser Ala
                   155                 160                 165
   Ser Val Trp Thr Glu Leu Leu Ser Arg Met Gly Arg Asn Asn Asp
                   170                 175                 180
   Arg Leu Ile Val Ala Ala Val Gly Asn Asp Gly Ala Asp Ile Arg
                   185                 190                 195
   Lys Leu Ser Ala Gln Gln Arg Ile Trp Pro Ala Ala Tyr His Pro
                   200                 205                 210
   Val Ser Ser Val Asn Lys Lys Gln Asp Pro Val Ile Arg Val Ala
                   215                 220                 225
   Ala Leu Ala Gln Tyr Arg Lys Gly Glu Thr Pro Val Leu His Gly
                   230                 235                 240
   Gly Gly Ile Thr Gly Ser Arg Phe Gly Asn Asn Trp Val Asp Ile
                   245                 250                 255
   Ala Ala Pro Gly Gln Asn Ile Thr Phe Leu Arg Pro Asp Ala Lys
                   260                 265                 270
   Thr Gly Thr Gly Ser Gly Thr Ser Glu Ala Thr Ala Ile Val Ser
                   275                 280                 285
   Gly Val Leu Ala Ala Met Thr Ser Cys Asn Pro Arg Ala Thr Ala
                   290                 295                 300
   Thr Glu Leu Lys Arg Thr Leu Leu Glu Ser Ala Asp Lys Tyr Pro
                   305                 310                 315
   Ser Leu Val Asp Lys Val Thr Glu Gly Arg Val Leu Asn Ala Glu
                   320                 325                 330
   Lys Ala Ile Ser Met Phe Cys Lys Lys Asn Tyr Ile Pro Val Arg
                   335                 340                 345
   Gln Gly Arg Met Ser Glu Glu Leu
                   350         353
<210> 20
   <211> 11
   <212> PRT
   <213> artificial sequence
   <223> linker sequence
<400> 20
<210> 21
   <211> 327
   <212> PRT
   <213> Escherichia coli
   <223> a fragment of Subunit A of subtilase cytotoxin
<400> 21

## Description

This invention relates to the cleavage of BIP (Immunoglobulin binding protein) by subtilase toxin and its application to inhibiting growth of or killing of cells. The invention has application to treatment of cancers and to conformational diseases and in particular to conformational diseases involving BiP that are influenced by cleavage by the subtilase cytotoxin. The invention also relates to subtilase toxin molecules joined to targeting moiety for targeting cells in particular tumor cells expressing epidermal growth factor receptors, or endothelial and tumor cells expressing vascular endothelial growth factor receptors.

### BACKGROUND OF THE INVENTION

The endoplasmic reticulum (ER) is a perinuclear, cytoplasmic compartment where proteins and lipids are synthesized. Within the ER reside molecular chaperones that facilitate proper protein folding, maintain proteins in a folded state, and prevent protein folding intermediates from aggregating. One of the best characterized ER chaperone proteins is BiP, also known as the glucose regulated protein 78 (GRP78). BiP was first discovered as a 78,000Da protein whose synthesis was enhanced in tissue culture cells grown in medium deprived of glucose. Subsequently, BiP was determined to be an ER resident protein and its synthesis can be stimulated by a variety of environmental and physiological stress conditions that perturb ER function and homeostasis. BiP is named because it was originally found to bind to the immunoglobulin heavy chains of pre-B cells.

BiP is a highly conserved ER chaperone essential for survival of eukaryotic cells⁶⁻⁸. It comprises an N-terminal ATPase and a C-terminal protein-binding domain. One of its functions is to mediate correct folding of nascent secretory proteins, by binding exposed hydrophobic domains via the C-terminus, with subsequent release coupled to N-terminal domain-catalysed ATP-hydrolysis^{7,8}. BiP is also responsible for maintaining the permeability barrier of the ER membrane by sealing the lumenal end of the Sec61 translocon pore, as well as for targeting of terminally mis-folded proteins to the Sec61 apparatus for degradation by the proteasome^{8,9}. BiP plays a crucial role in the unfolded protein response (UPR) as the ER stress-signalling master regulator⁷. For survival under ER stress, the UPR shuts down general protein translation while selectively activating expression of chaperone proteins such as BiP, which exhibits anti-apoptotic properties through interference with caspase activation and probably other yet undefined mechanisms. Thus BiP induction under pathological conditions may represent a major cellular protective mechanism for cells to survive ER stress and could have implications in organ preservation as well as cancer progression. BiP also exhibits anti-apoptotic properties through interference with caspase activation. Thus, disruption of BiP function has inevitably fatal consequences for the cell^{7,10-12}.

Protein misfolding is implicated in the pathogenesis of many genetic diseases. Missense mutations and small in frame deletions or insertions rarely affect the function of a given protein directly. Mostly these disease causing mutations affect the ability of the proteins to fold into a correct conformation, and they often give rise to premature degradation or aggregation of the mutant proteins. Diseases caused by this kind of molecular pathological mechanism are termed "conformational diseases".

Anti sense BiP has been used to study conformational diseases and ER stress related matters. A problem with use of antisense is however that it appears that compensatory mechanisms come into play, so that the levels of BiP do no fall away as much as hoped ⁴³. Additionally a problem is that antisense does not inactivate existing and active BiP. Anti-BiP specific antibody is also available however exogenous antibody does not provide a means of inactivating intracellularly acting molecules such as BiP.

Subtilase cytotoxin (SubAB) was discovered in a highly virulent O113:H21 Shiga toxigenic *Escherichia coli* (STEC) strain, which was responsible for an outbreak of haemolytic uraemic syndrome (HUS) in South Australia in 1998, and has since been detected in several other STEC serotypes^{2,4}. SubAB is more toxic for Vero (African green monkey kidney) cells than Shiga toxin, and is lethal for mice, resulting in extensive microvascular damage, thrombosis and necrosis in multiple organs, including the brain, kidneys and liver². It was named "Subtilase cytotoxin", because its 35-kDa A subunit (SubA) shares sequence homology to a subtilase-like serine protease of *Bacillus anthracis²,* although the sequence identity was low (26%). Mutagenesis of the critical Ser₂₇₂ residue in SubA to Ala abolished *in vitro* and *in vivo* toxicity. Thus, serine protease activity is central to the mechanism of action of the holotoxin². Subtilase cytotoxin is disclosed in WO2005061714.

For the purpose of this specification the word "comprising" means "including but not limited to", and the word "comprise" has a corresponding meaning. Also a reference within this specification to a document is not to be taken as an admission that the disclosure therein constitutes common general knowledge in Australia.

### SUMMARY OF THE INVENTION

The invention arises from the finding that SubA of the subtilase toxin cleaves bovine BiP at position 416 and 417 (between two leucine residues) to inactivate BiP. Proteomic analysis shows that the cleavage is quite specific with no other significant cleavage of other proteins occurring. BiP as indicated above plays an important role in the conformation of a range of proteins, and its deficiency has been linked with a number of conformational diseases. This has implications for its use in cellular and animal models, that may assist with the elucidation of conformational diseases. BiP also plays an important role in the protective Endoplasmic Reticulum(ER) stress in response to a variety of environmental and physiological stress conditions, including various therapeutic treatments, and the present invention being able to specifically remove BiP also provides an avenue for exploiting ER stress in rational development of therapeutic treatments. Cleavage of BiP also has specific application in treating cancers in particular solid tumors. The invention also provides for targeted inhibition of growth or cytotoxicity.

The invention provides the use of SubA or an active variant thereof in the manufacture of a medicament for the treatment of tumors.

For a better understanding the invention will now be described by reference to examples and drawings as follows:

### BRIEF DESCRIPTION OF THE DRAWINGS

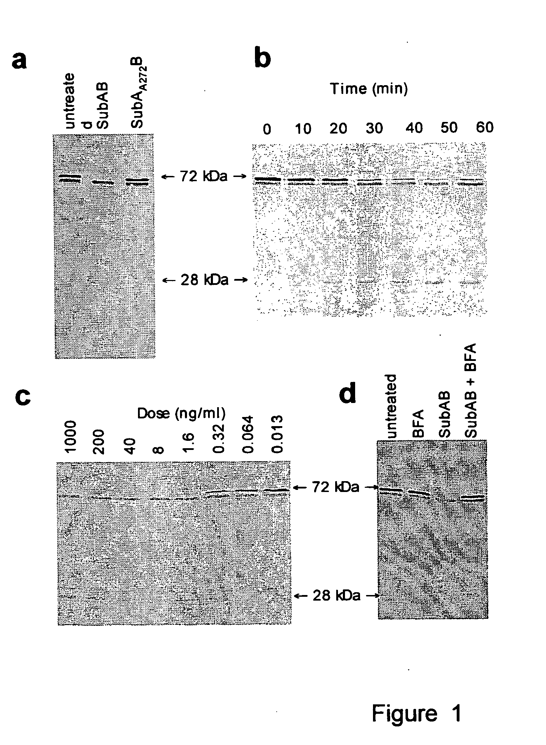

- Figure 1: SubAB cleaves BiP in Vero cells. Vero cell monolayers in 24 well plates were incubated at 37°C in DMEM growth medium with or without purified SubAB or SubA_{A272}B, as indicated. Cell monolayers were then solubilized and subjected to SDS-PAGE and Western blot analysis using polyclonal goat anti-BiP (see Methods). **a**, Cleavage of BiP by 1 µg/ml SubAB or SubA_{A272}B after 3 h incubation. **b**, Time course of BiP cleavage at 1 µg/ml SubAB. **c,** Dose response experiment; cells were treated with the indicated dose of SubAB for 60 min. **d,** Effect of brefeldin A (BFA) on BiP cleavage; cells were pre-treated for 30 min with or without 0.5 µg/ml BFA, and then treated with or without 1 µg/ml SubAB for 60 min.

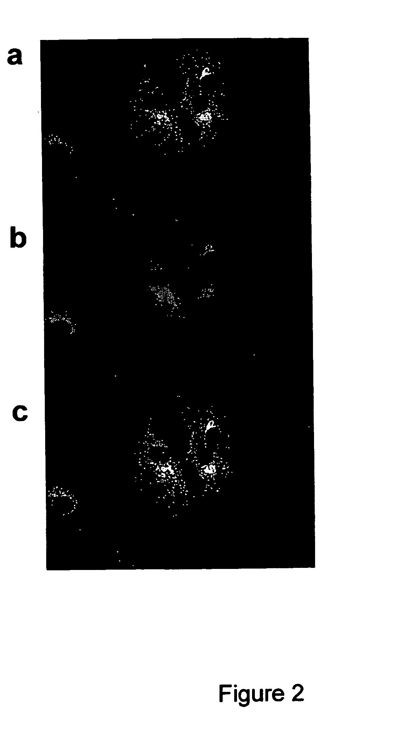

- Figure 2: Co-localization of SubAB and BiP. Vero cells growing on coverslips were treated with 1 µg/ml Oregon Green-SubAB for 60 min, excess toxin was then removed and cells were incubated in fresh medium for 30 min. Cells were then fixed, permeabilized and reacted with goat anti-BiP (C-20), followed by Texas Red- (TR)- conjugated rabbit anti-goat IgG. Washed coverslips were examined by confocal microscopy. **a,** Oregon Green channel. **b**, Texas red channel. **c**, overlaid images.

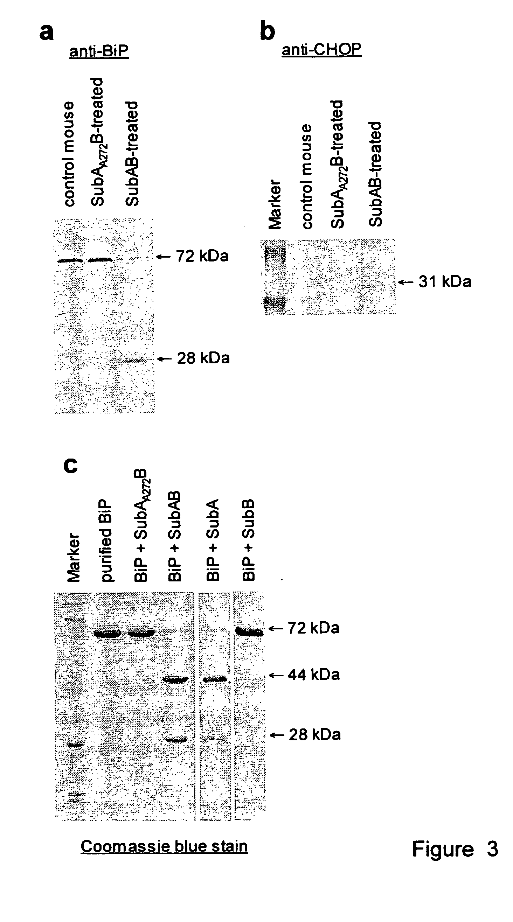

- Figure 3: **a,** *In vivo* cleavage of murine BiP. Liver tissue from a normal mouse, or from mice 24 h after intraperitoneal injection with 5 µg SubAB or SubA_{A272}B was separated by SDS-PAGE and subjected to Western blot analysis using polyclonal goat-anti-BiP (see Methods). **b**, *In vivo* induction of CHOP. Liver tissue samples were also subjected to Western blot analysis using monoclonal anti-CHOP (9C8; Alexis Corporation, Lausen, Switzerland). The expected mobility of CHOP (31 kDa) is indicated; Marker track shows 38-kDa and 26-kDa bands of Benchmark™ Prestained Protein Ladder. **c**, *In vitro* cleavage of purified BiP. Purified bovine BiP (5 µg) (Sigma) was incubated in DMEM with or without 100 ng SubAB, SubA_{A272}B, SubA alone, or SubB alone, for 1 h at 37° C in a volume of 15µl Proteins were then separated by SDS-PAGE and stained with Coomassie blue. Fragment sizes were determined with reference to the Markers (BioRad "broad range").

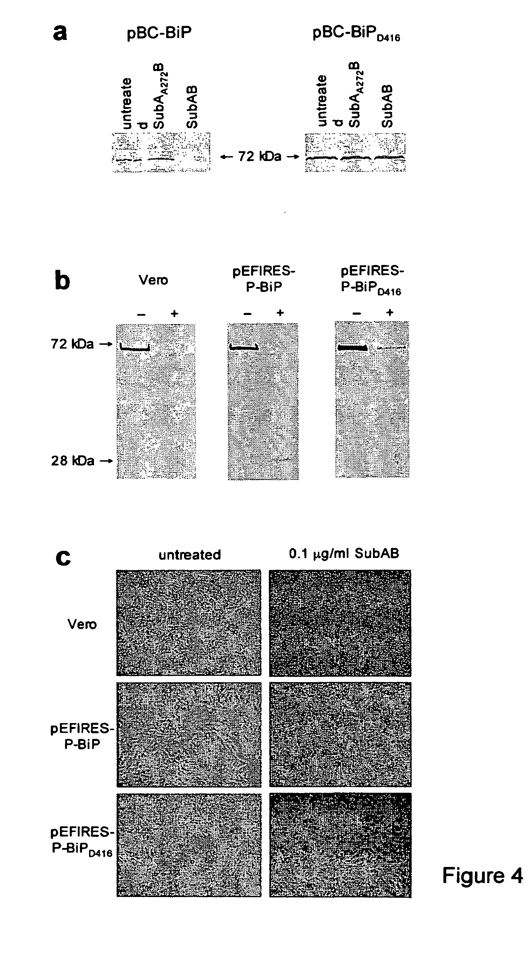

- Figure 4: **a,** SubAB cleavage of BiP and BiP_{D416} expressed by recombinant *E*. *coli.* Lysates of *E. coli* JM109 carrying pBC-BiP or pBC-BiP_{D416} were treated with or without 1 µg/ml SubAB or SubA_{A272}B for 1 h at 37° C, and then subjected to SDS-PAGE and Western blot analysis using polyclonal goat anti-BiP. The mobility of undigested BiP (72 kDa) is indicated. **b**, Cleavage of BiP in transfected Vero cells. Untransfected Vero cells, or cells transfected with pEFIRES-P-BiP or pEFIRES-P-BiP_{D416} were treated with (+) or without (-) 1 µg/ml SubAB for 2 h at 37° C, and then subjected to SDS-PAGE and Western blot analysis as above. **c**, Cytotoxicity of SubAB for transfected cells. Cell monolayers were exposed to 0.1 µg/ml SubAB and cytotoxicity was assessed after 3 days at 37° C.

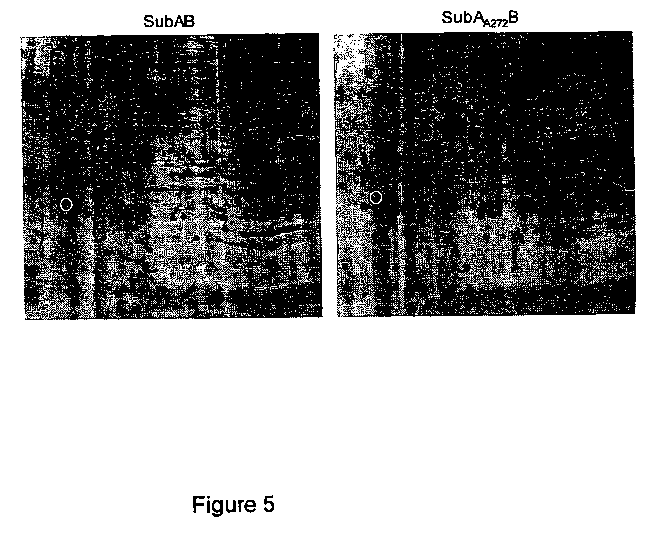

- Figure 5: Silver-stained 2D PAGE of proteins from Vero cells treated with SubAB or SubA_{A272}B. The IEF dimension is horizontal with acidic pI on the left. The mobility of protein size markers in the vertical dimension is indicated on the right hand side of each image. The IEF internal standard tropomyosin shows two polypeptide spots of similar pI; the lower spot of MW 33,000 and pI 5.2 is marked with a black arrowhead. The location of the SubAB-specific spot #666 (subsequently identified as BiP) and the equivalent position on the SubA_{A272}B gel is circled in white.

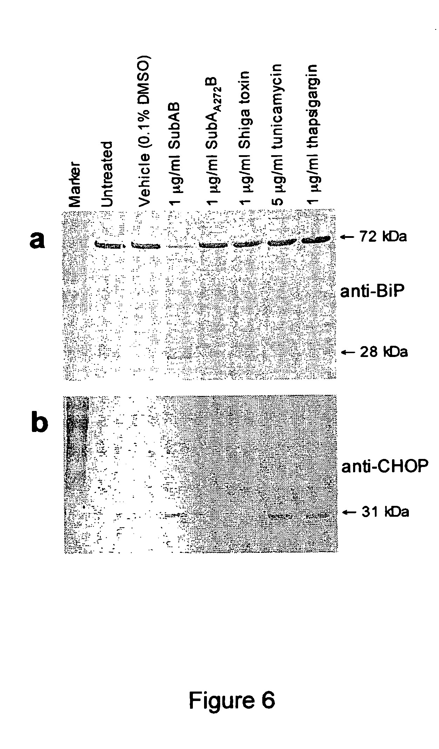

- Figure 6: Cleavage of BiP and induction of an unfolded protein response (UPR) in N2A (murine neuroblastoma) cells. N2A monolayers were treated with either SubAB, SubA_{A272}B, purified Shiga toxin, or the known ER stressors tunicamycin and thapsigargin, for 24 h, as indicated. Cell lysates were then subjected to Western blot analysis using: **a,** anti-BiP C-20, or **b,** monoclonal anti-CHOP (9C8; Alexis Corporation, Lausen, Switzerland).

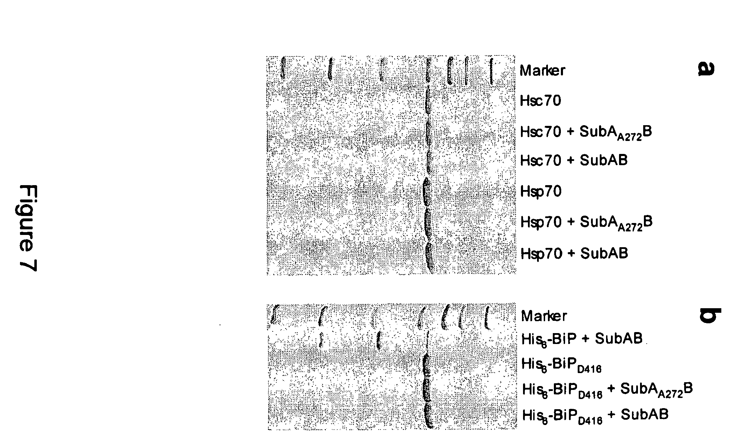

- Figure 7: Cleavage of purified *chaperones in vitro.* **a,** Purified Hsc70 or Hsp70 (5 µg per reaction) (obtained from Stressgen, Victoria, BC, Canada) was incubated in DMEM with or without 100 ng SubAB or SubA_{A272}B in a volume of 10 µl. Proteins were then separated by SDS-PAGE and stained with Coomassie blue. **b**, Native murine BiP or BiP_{D416} was expressed in recombinant *E. coli* using the vector pQE30 (Qiagen, Germany) such that a His₆ tag was fused to the N-terminus (minus the signal peptide). The proteins were purified from the recombinant *E. coli* lysates by Ni-NTA chromatography under non-denaturing conditions in accordance with the manufacturer's instructions. Purified His₆-BiP or His₆-BiP_{D416} (5 µg per reaction) was then incubated with 100 ng SubAB or SubA_{A272}B, and separated by SDS-PAGE as above. Markers are BioRad "broad range"(sizes 200, 116, 97, 66, 45, 31 and 21 kDa).

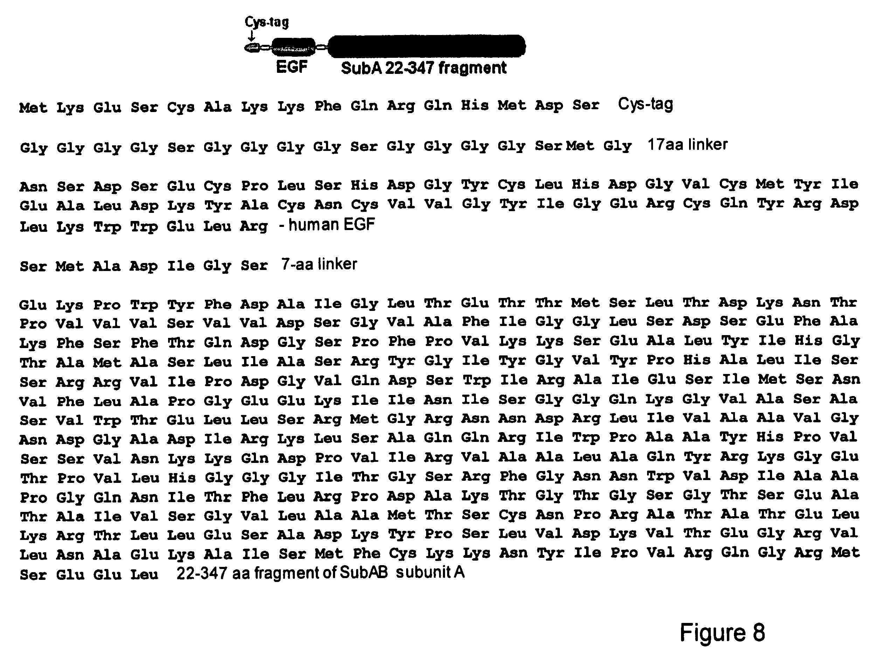

- Figure 8: is a schematic representation of the EGF-SubA fusion protein. EGF-Sub A comprises an N-terminal cystein-containing Cys-tag (SEQ ID NO:10) (described earlier ^{26,27,28,29,30}) for site-specific modification, complete sequence of human epidermal growth factor (SEQ ID NO:9), and a fragment of SubAB subunit A without a leader sequence (SEQ ID NO:8). The complete amino acid sequence of EGF-SubA fusion protein (SEQ ID NO:13) has 419 amino acids and exact mass, as calculated on the basis of amino acid sequence, of 44948.4 daltons. The complete EGF-SubA fusion protein comprises a first linker sequence (SEQ ID NO:11) of 17 amino acids and a second linker sequence (SEQ ID NO:12) of 7 amino acids.

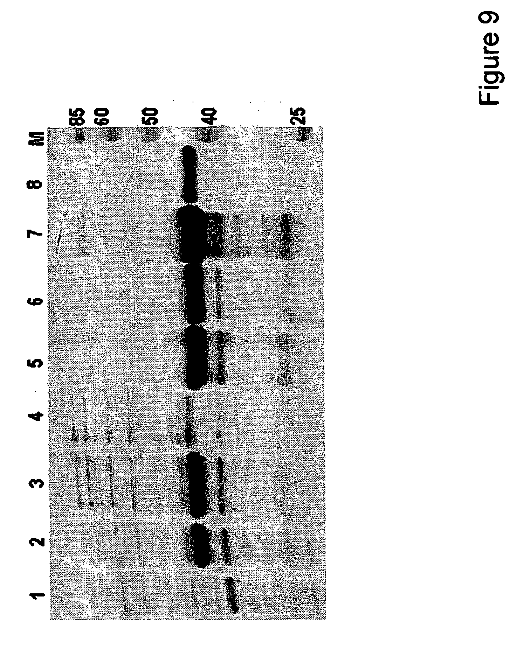

- Figure 9: illustrates expression and purification of EGF-SubA from BL21(DE3) *E. coli* strain. Expression of EGF-SubA was induced by addition of isopropyl-β-D-thio-galactopyronoside (IPTG) to a final concentration of 1 mM (lane 1, no induction; lane 2, IPTG-induced culture). BL21(DE3) cells were harvested after 2 hours of IPTG induction and homogenized by high-pressure homogenization. Bacterial homogenate (Fig. 9 lane 3) was separated to soluble (Fig. 9 lane 4) and insoluble (Fig. 9 lane 5) parts by centrifugation. EGF-SubA accumulated in insoluble part of bacterial lysate was solubilized in 8M urea (Fig. 9 lane 6). Fig 9 also illustrates the quality of EGF-SubA refolded from inclusion bodies (Fig. 9 lane 7) and its final preparation (Fig. 9 lane 8) after ion-exchange chromatography. All samples were analyzed by reducing SDS-PAGE on 15% gels followed by SafeBlue staining (Invitrogen). Molecular weights of pre-stained protein markers (Protein Ladder, BioRad laboratories, USA) are indicated in kDa (Fig. 9 lane M). Fig 9 illustrates that, in accordance with the calculated molecular mass of 44948.4 daltons, purified EGF-SubA fusion protein migrates as a polypeptide with an apparent molecular weight above 40 kDa under conditions of reducing SDS-PAGE.

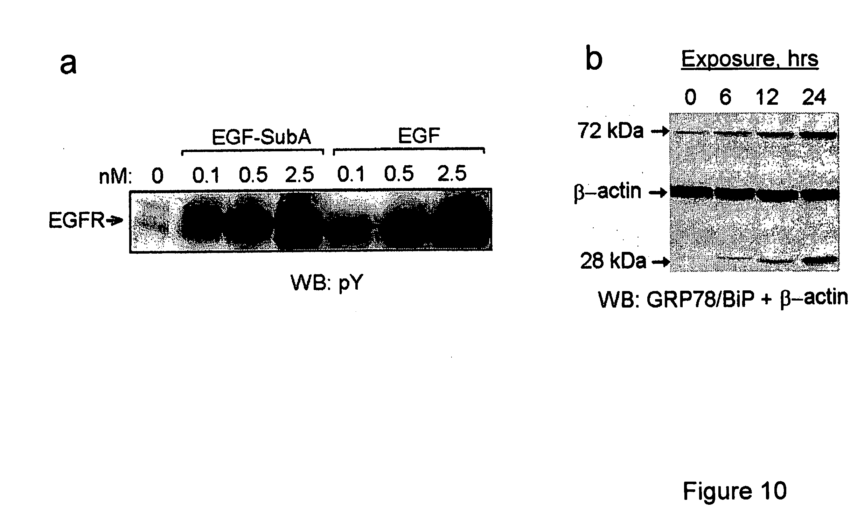

- Figure 10: illustrates functional activity of both EGF and SubA domains of EGF-SubA fusion protein in tissue culture. EGF-SubA induced EGFR tyrosine autophosphorylation in rat glioma cells overexpressing EGF receptor EGFR (F98/EGFR cells, Fig. 10A). Tyrosine phosphorylation of EGFR was detected by Western blot analysis of the lysates of F98/EGFR cells treated with EGF-SubA or control recombinant human EGF (Sigma), using anti-phosphotyrosine antibody (Sigma). Similar activity of EGF-SubA and control EGF in the same concentration range indicates that fusion of N-terminal Cys-tag and C-terminal SubA did not affect significantly the ability of EGF to bind and activate its receptor EGFR. Figure 10 also illustrates that EGF-SubA selectively cleaves intracellular BiP in MDA231luc cells (a luciferase-expressing derivative of EGFR-positive human breast carcinoma cells MDA-MB-231), thereby confirming that EGF-SubA fusion protein retains the substrate specificity of the parental SubAB toxin (Fig 10B). MDA231luc cells were grown on 6-well plates to near-confluence, exposed to 1 nM EGF-SubA for indicated periods of time, then lyzed and clarified by centrifugation. Clarified cell lysates were analyzed by Western blotting with BiP-specific antibody (Santa Cruz, USA) and control β-actin-specific antibody (Sigma) to ensure equal protein loading (Fig. 10B). Up-regulation of a 72-kDa band corresponding to intact BiP and the appearance of its 28-kDa cleavage fragment was detected after a 6-h exposure of MDA231luc cells to 1 nM EGF-SubA.

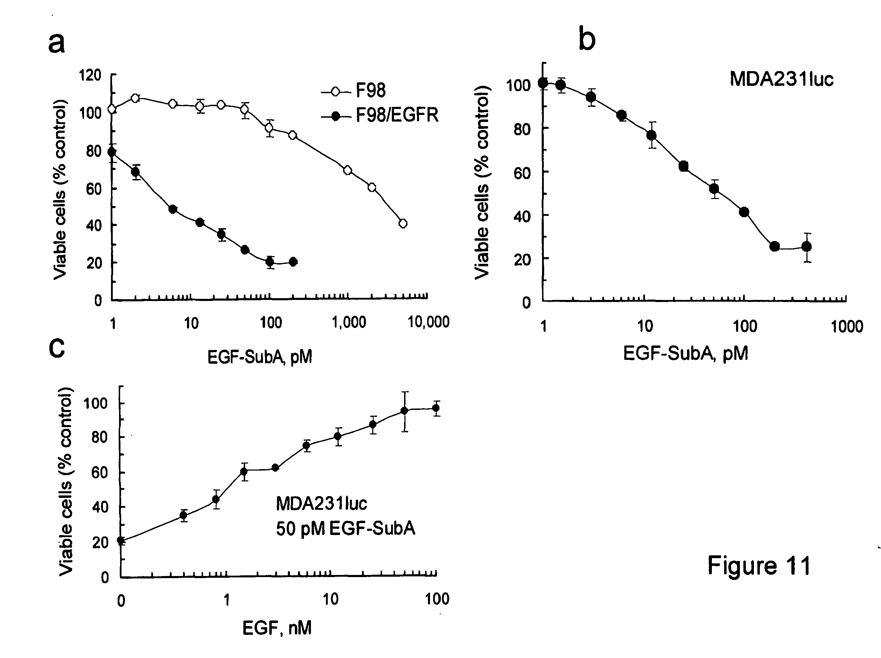

- Figure 11: illustrates that EGF-SubA is selectively cytotoxic toward F98/EGFR cells (IC₅₀ of 5 pM, Fig. 11A). Control parental F-98 cells lacking EGF receptors were ∼500-fold less sensitive (IC₅₀ of ∼2.5 nM), thus confirming selective targeting of SubA moiety into EGFR-expressing cells by EGF moiety of EGF-SubA fusion protein. Figure 11 also illustrates EGF-SubA toxicity toward MDA-231 luc cells with an IC₅₀ of ∼50 pM (Fig. 11B), and this toxicity was inhibited by excess of EGF (Fig. 11 C), confirming its EGFR-dependent mechanism.

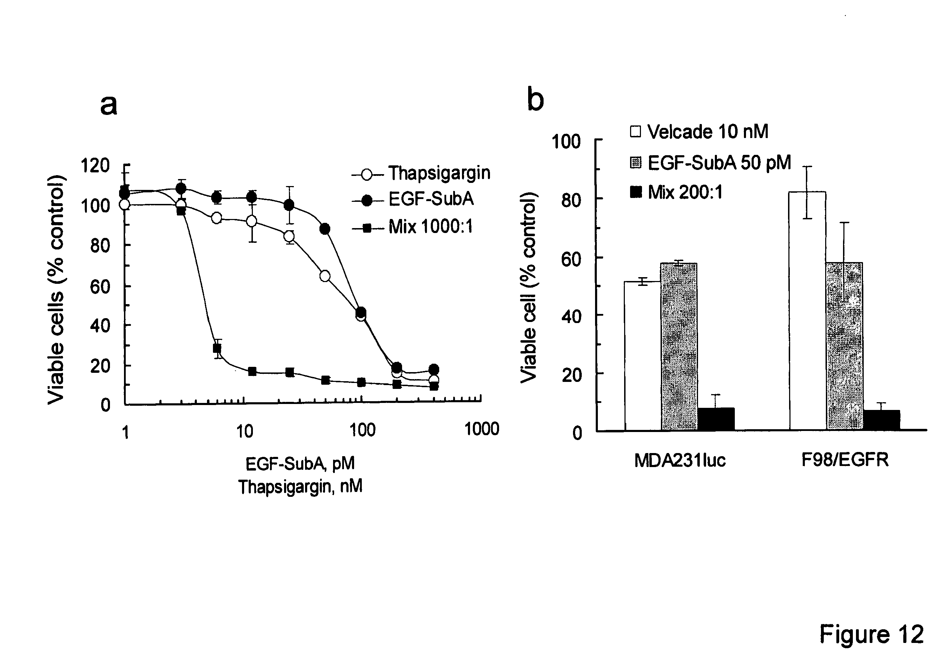

- Figure 12: illustrates that EGF-SubA enhances cell growth inhibition induced by an ER stress inducer thapsigargin (Sigma), and a proteosome inhibitor bortezomib (Velcade, Millenium Pharmaceutical). MDA231luc cells plated onto triplicate wells of 96-well plates at a density of 2,000 cells/well were exposed to varying amounts of thapsigargin (Fig. 12A) and bortesomib (Fig. 12B), either alone or in combination with EGF-SubA. Viable cells were quantitated after 72 hours of exposure using Cell Proliferation Assay kit (Promega). Viable cell in untreated control wells were taken as 100%. For each experimental condition, average values and standard deviations are presented.

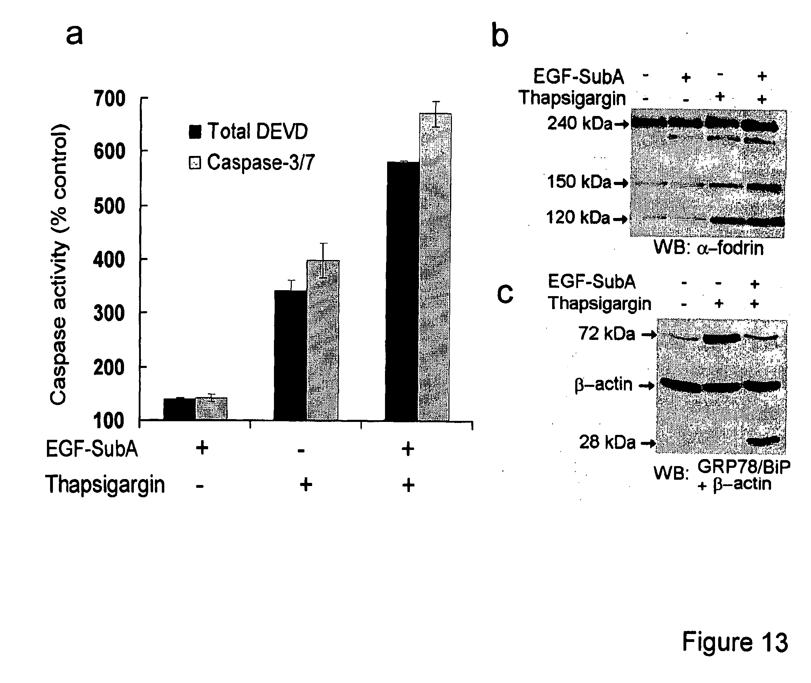

- Figure 13: illustrates that EGF-SubA enhances thapsigargin-induced apoptosis in MDA231luc cells. MDA231luc cells plated onto triplicate wells of 6-well plates at a density of 500,000 cells/well were exposed to EGF-SubA and thapsigargin, either alone or in combination, 18 hrs later. After 24 hrs of exposure, cells were lysed and separated to nuclear and cytosol fractions. Cytosol aliquots containing equal amounts of protein (40 µg per assay) were analyzed by enzymatic caspase activity assay³⁰ and Western blot analysis. A significant increase in total DEVD- and specific caspase-3/7 activity was detected in lysates of cells exposed for 24 hours to EGF-SubA and thapsigargin combination, as compared to treatment with each drug alone for the same period of time (Fig. 13A). Western blotting with BiP-specific and β-actin-specific antibody (Fig 13B) was done as described in legend to Fig. 11. Figure 13B shows enhanced expression of BiP in cells treated with thapsigargin alone (Fig. 13B, lanes 2-4) and effective cleavage of BiP with EGF-SubA in combination treatment regimen (Fig. 13B, lanes 5-7). Control β-actin-specific antibody confirmed equal protein loading. Western blot analysis with α-fodrin-specific antibody (Fig. 13C) was done as earlier described³¹. Greater accumulation of 150-kDa and 120-kDa fragments of α-fodrin after exposure to EGF-SubA and thapsigargin combination treatment (Fig. 13C) confirmed data on enhanced apoptosis due to such treatment obtained with caspase enzymatic activity assay (Fig. 13A).

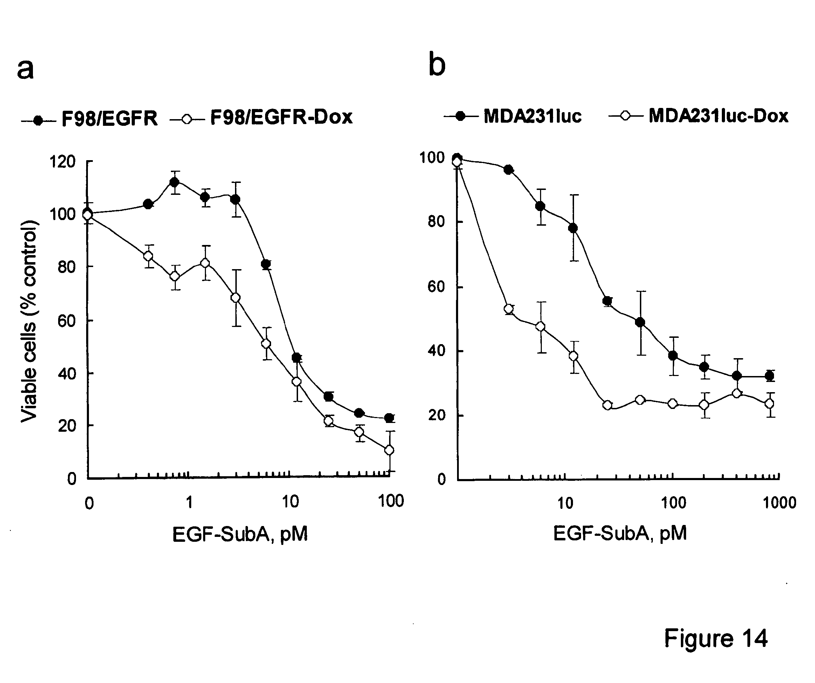

- Figure 14: illustrates that doxorubicin-resistant MDA231luc-Dox and F98/EGFR-Dox derivatives of MDA231luc and F98/EGFR cells become significantly more sensitive to EGF-SubA than respective parental cells. MDA231luc-Dox and F98/EGFR-Dox were derived from the corresponding parental cells and maintained in 75-cm² T-flasks in the presence of 20 nM and 200 nM doxorubicin, respectively, for 8-10 weeks. Doxorubicin-resistant and parental cells were plated onto triplicate wells of 96-well plates at a density of 2,000 cells/well were exposed to varying amounts of EGF-SubA. Viable cells were quantitated after 72 hours of exposure using Cell Proliferation Assay kit (Promega). Viable cells in untreated control wells were taken as 100%. For each experimental condition, average values and standard deviations are presented.

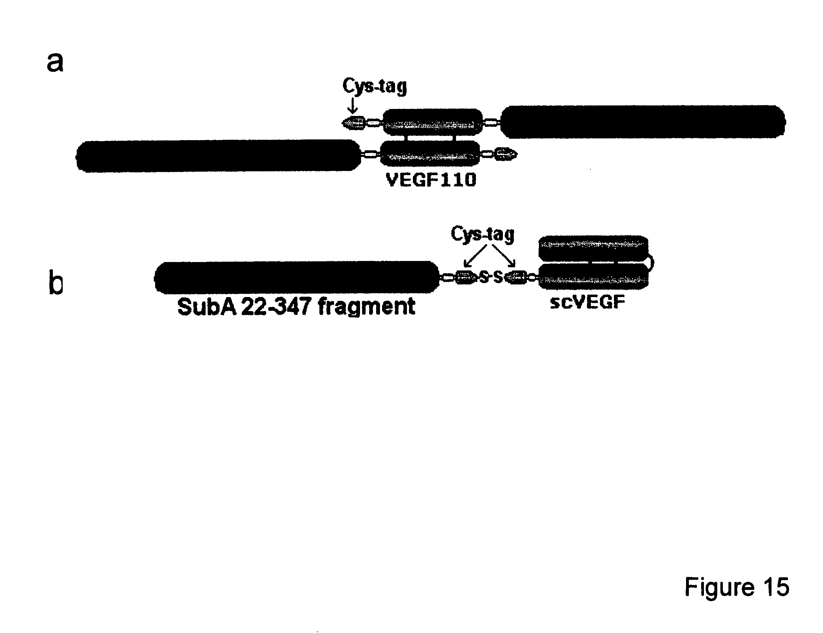

- Figure 15: illustrates construction of proteins that combine SubA moiety and vascular endothelial growth factor, using two different methods. Figure 15A illustrates construction of VEGF-SubA fusion protein by genetically fusing SubA moiety to the C-terminus of a truncated version of VEGF₁₂₁ described by Blankenberg *et al.* (2006)*³³*. VEGF-Sub A is a homodimeric protein in which each subunit comprises an N-terminal cystein-containing Cys-tag (SEQ ID NO:10) for site-specific modification, a 3-112 aa fragment of a 121 aa version of human vascular endothelial growth factor (VEGF₁₂₁) (SEQ ID NO:16), and a fragment of SubAB subunit A without a leader sequence (SEQ ID NO:8). The complete amino acid sequence of a subunit of homodimeric VEGF-SubA fusion protein (SEQ ID NO:17) has 464 amino acids and exact mass, as calculated on the basis of amino acid sequence, of 50,665.3 daltons. The complete amino acid sequence of a subunit of homodimeric VEGF-SubA fusion protein comprises a first linker sequence (SEQ ID NO:18) of 5 amino acids and a second linker sequence (SEQ ID NO:12) of 7 amino acids.
- Figure 15B: illustrates construction of VEGF-S-S-SubA protein conjugate by conjugating via site-specific disulfide bond formed between a recombinant Cys-tagged SubA (SEQ ID NO:19) and recombinant Cys-tagged scVEGF (SEQ ID NO:21), a single-chain VEGF, comprising two 3-110 fragments of VEGF₁₂₁ fused head-to-tail as earlier described²⁷. Cys-tagged SubA (SEQ ID NO:19) comprises an N-terminal cystein-containing Cys-tag (SEQ ID NO:10) for site-specific modification, a linker sequence (SEQ ID NO:20) of 11 amino acids, and a fragment of SubAB subunit A without a leader sequence (SEQ ID NO:8). The complete amino acid sequence of a Cys-tagged SubA (SEQ ID NO:19) has 353 amino acids and exact mass, as calculated on the basis of amino acid sequence, of 37,712.3 daltons.

### DETAILED DESCRIPTION OF THE ILLUSTRATED AND EXEMPLIED EMBODIMENTS OF THE INVENTION

### General definitions

By way of a shorthand notation the following three and one letter abbreviations for amino acid residues are used in the specification as defined below.

| Amino Acid | Three-letter Abbreviation | One letter Abbreviation |
|---|---|---|
| Alanine | Ala | A |
| Arginine | Arg | R |
| Asparagine | Asn | N |
| Aspartic Acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

Where a specific amino acid residue is referred to by its position in the polypeptide of an protein, the amino acid abbreviation is used with the residue number given in subscript (i.e. Xaaₙ).

"Subtilase" and "Subtilase cytotoxin" refers to a holotoxin having an AB₅ structure described in WO2005061714. The holotoxin thus consists of a catalytic A subunit (SubA) of an approximate molecular weight of 35kDa and 5 B subunits (sub B) of an approximate molecular weight of 13kDa each. SubA is shown herein to cleave bovine BiP between two leucine residue at positions 416 and 417. SubB plays a role in binding of the toxin to target cells and transporting SubA intracellularly.

BiP herein referred to as BiP (Immunoglobulin Binding Protein) is a chaperone molecule active in the Endoplasmic Reticulum, and consists of an ATPase domain and a protein binding domain with a hinge between the two domains.

Reference made herein to "two adjacent hinge leucine residues" are residues that are in a putative hinge region between the ATPase domain and the protein binding domain of BiP. The two amino acids are at positions 416 and 417 in bovine BiP (accession no. P11021). These are the two amino acids between which cleavage occurs with SubA.

The BiP molecule may be derived from any one of a number of species, including a rodent, (mouse, rat or ferret) agricultural animal, (sheep, cow, goat, rabbit) including poultry (chicken, ducks, turkeys) companion animal (cat, dog) competition animal (horse). The BiP molecule may also be a variant of any one of these, including having amino acid substitution deletion or other rearrangement and including a chimeric protein that still comprises the hinge region and having the two adjacent hinge leucine residues intact.

Active variants of SubA encompass a protein that retains the catalytic action of cleaving between the two adjacent hinge leucine residues, but differs from the wild type SubA. The variation may be minor and constitute conservative substitutions apart from at the critical Ser₂₇₂, Asp₅₂ or His₈₉ residues. Conservative substitutions are designed to alter one amino acid for another similar acting amino acid. Typical substitutions are those made in accordance with the following:
Suitable residues for conservative amino acid substitutions

| Original Residue | Exemplary Substitutions |
|---|---|
| Ala | Ser |
| Arg | Lys |
| Asn | Gln; His |
| Asp | Glu |
| Cys | Ser |
| Gln | Asn |
| Glu | Asp |
| Gly | Ala |
| His | Asn; Gln |
| Ile | Leu; Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

Non conservative substitutions may also be made at non-sensitive locations. Rearrangements may also be made and still retain activity, and thus include deletions, additions, inversions and duplications. Similarly the active variant may also comprise a chimeric protein between SubA or a fragment thereof and another protein. An active variant can readily be tested for example either by Vero cell toxicity or alternatively cleavage of BiP.

Specific active variants may include fusion proteins, that may comprise SubA or an active fragment thereof and further comprise a receptor or an antigen binding moiety, the receptor or antigen binding moiety being capable of binding a receptor or an antigen exposed on the surface of a target cell.

Specific active variants may include protein conjugates, that may comprise SubA or an active fragment thereof and further comprise a receptor or an antigen binding moiety, the receptor or antigen binding moiety being capable of binding a receptor or an antigen exposed on the surface of a target cell.

Disclosed is a fusion protein between epidermal growth factor and SubA (EGF-SubA). The EGF-SubA fusion protein includes full-length human epidermal growth factor (EGF) that binds to EGF receptors and full-length A subunit of SubAB bacterial toxin without a leader sequence that confers proteolytic activity directed against BiP. The EGF-SubA fusion protein preferably has an N-terminal cysteine containing tag (Cys-tag^{27,28,29,30,32,} US patent application 20050221431) for site-specific conjugation to EGF-SubA of various entities, including but not limited to modifying agents such as polyethyleneglycol or phospholipid, and agents for imaging in different modalities, such as near-infrared fluorescent dyes or radionuclide chelators.

The nucleic acid sequences for the SubAB toxin and the EGF are individually known in the art. However, the inventors have surprisingly found that a combination of these two sequences provides for production of a fusion protein with a unique combination of characteristics. The fusion protein is capable of binding to specific EGF cellular receptor by virtue of the EGF domain and undergo receptor-mediated internalization. The fusion protein is also capable of cleavage of intracellular protein BiP and growth inhibition in cells expressing EGF receptors by virtue of the SubAB toxin domain. In combination, these two protein domains provide an effective and highly targeted treatment of pathophysiological conditions that involves cells subjected to signaling through interaction of epidermal growth factor and its cognate receptors, such as cells in many primary tumors and metastasis. In addition, the proteins and pharmaceutical compositions of the present invention may be used either alone, or in combination with other treatments, particularly treatments to which targeted cells defensively respond with enhanced expression of BiP, including but not limited to treatment with drugs, which directly induce stress in ER by affecting Ca2+ content in ER or by inhibiting proteasome activity, or protein glycosylation, or by inducing oxygen or glucose deprivation, or drugs whose efficacy is decreased because of development of BiP dependent drug resistance. In fact, the unique mechanism of action of EGF-SubA provides a rational basis for designing combination regimens, whereby EGF-SubA destroys the cellular BiP-based defences against various drugs.

Disclosed herein is the provision of a recombinant expression vector harboring the new DNA sequence and transformed bacterial cells containing such recombinant expression vector. The nucleic acid sequence coding for the fusion protein EGF-SubA may be inserted into known vectors, such as other bacterial, viral, yeast, or mammalian expression vector, using materials and methods well known in the art. The nucleic acid construct coding for the EGF-SubA fusion proteins is inserted into a plasmid such that nucleic acid construct is operatively linked to an inducible promoter sequence, a sequence that encodes Cys-tag that enable site-specific modification with functionalized SH-directed moieties, such as polyethylenglycols of various length for improving pharmacokinetic of EGF-SubA and decreasing host immune response to EGF-SubA, or contrast agents for various modalities for monitoring EGF-SubA in vivo. The plasmid is also preferably introduced into a host cell, such as a bacterial cell, in which the promoter is inducibly regulated.

The EGF-SubA fusion protein is useful as an agent that targets and inhibits growth of EGFR-expressing cells and thereby is useful for treating certain diseases, including, but not limited to, solid tumor and metastasis growth. As used herein, to target EGF-SubA fusion protein means to direct it to cells that express EGF receptors. Upon binding to the receptor EGF-SubA fusion protein is internalized by the cell via receptor-mediated endocytosis and induces cell growth inhibition and death.

As used herein, the term active, or reference to the activity of EGF-SubA fusion protein or its cytostatic and cytotoxic effects, refers to the ability of such protein to exert SubA serine protease activity, upon EGF-receptor mediated internalization of EGF-SubA fusion protein by the cells. Such activity may be assayed *in vitro* and *in vivo* by any method known to those of skill in the art including, but not limited to, the assays that measure receptor binding, autophosphorylation and internalization and assays that assess cytoxic and cytostatic effects by measuring the effect of a test compound on cell proliferation and cell death.

As used herein, EGF refers to polypeptides having amino acid sequences of native EGF proteins, as well as modified sequences, having amino acid substitutions, deletions, insertions or additions of the native protein but retaining the ability to bind to EGF receptors and to be internalized.

It is understood that differences in amino acid sequences can occur among EGFs of different species as well as among EGFs from individual organisms or species. Reference to EGFs is also intended to encompass proteins isolated from natural sources as well as those made synthetically, as by recombinant means or possibly by chemical synthesis. EGF also encompasses mutants of EGF that possess the ability to target SubA to EGF-receptor expressing cells and created in order to, for example, retain or increase the activity or stability of the growth factor, to reduce or eliminate disulfide scrambling, or to alter reactivity with various modifying groups (e. g. polyethylene glycol).

As used herein, the term "EGF receptor" is used to refer to receptors that specifically interact with EGF and transport it into the cell.

As used herein, the term "polypeptide reactive with the EGF receptor" refers to any polypeptide that specifically interacts with EGF receptor and is transported into the cell by virtue of its interaction with the EGF receptor.

Unless defined otherwise, all additional technical and scientific terms used herein have the same meaning as is commonly understood by one of skill in the art to which the subject matter herein belongs.

Fusion protein denoted EGF-SubA consisting of EGF linked to SubA inhibit growth of F98/EGFR rat glioma cells overexpressing EGF receptor EGFR in a dose-dependent manner with IC50 of ∼5 pM and MDA231luc breast carcinoma cells, expressing ∼10-fold less EGFR/cell, with IC50 of ∼50 pM. These effects depend on the ability of EGF moiety to bind to EGFR, because parental F98 cells lacking EGFR are ∼500-fold less sensitive to EGF-SubA (IC50 of ∼2.5 nM) and excess of free EGF rescue cells from EGF-SubA-induced toxicity. The results demonstrate that at picomolar concentration EGF-SubA can enter cells via EGF receptors, and SubA moiety of said molecule can effectively cause cytotoxic and/or cytostatic effects in cells overexpressing EGF receptors, but not in cells that have no receptors.

These results demonstrate the possibility of using EGF-SubA protein to target selectively a subpopulation of aggressively growing cancer cells known to overexpress EGF receptors, with significantly smaller effect on cells with physiologically normal levels of EGF receptors, thereby minimizing undesired side effects that might arise from interaction with not-targeted cells.

The compositions for use in inhibition of cell growth in vivo comprise a fusion protein in combination with a pharmaceutically acceptable diluent or carrier. The compositions according to the invention will in practice normally be administered by intravenous injection, continuous infusion, although other methods, such as parenternal injection or intramuscular injection may also be used. Compositions for injection can be provided in unit dose form and can take a form such as solution and can contain formulating agents, such as stabilizing agents, buffers, and the like.

Since EGF-SubA inactivates BiP it is reasonable to expect that this protein will be particularly effective against cells that rely on enhanced expression of BiP in their protective response against chemical or environmental insults (reviewed recently ^{42,37,41. 38}). Such treatments include but are not limited to insults that induce conditions known as ER stress and unfolded protein response. Such insults known in the art include, but are not limited to inhibitors of Ca2+ -dependent ER ATPase, such as thapsigargin and inhibitors of proteasome such, as bortezomib inhibitors of glycosylation, such as tunicamycin, drugs that induce oxygen and/or glucose depravation, such as anti-vascular agents combretastatin A4P and contortrostatin³⁶, and chemotherapeutic drugs, whose efficacy is decreased because of the development of BiP dependent drug resistance. As was recently established in art, the expanding list of such chemotherapeutic drugs includes, but is not limited to etoposide, doxorubicin, camptothecin, and vincristine³⁹, fudarabine³⁴, the retinoid analog, fenretinide³⁵, and non-coxib analogue of celecoxib⁴⁰.

Our results demonstrate that combination of such drugs with EGF-SubA produce a remarkably synergistic toxic effect observed at doses of both components that are far below respective EC50 values. In fact, a specific molecular endpoint of EGF-SubA action, cleavage of BiP allows for rational development of combination regimens, when this endpoint is used for development of optimized timing and doses of individual components.

Using methods known in art, it is also possible to combine SubA with other targeting proteins, making either fusion proteins comprising SubA and targeting protein, or protein conjugates that chemically and/or physically link SubA and targeting protein into a single entity and than use such fusion proteins or protein conjugates for targeting cells expressing specific receptors and/or antigens to achieve targeted inactivation of BiP, which might be beneficial by itself or in combination with other treatments to which cell respond with enhanced expression of BiP.

Suitable targets known in art include but are not limited to receptors that are expressed at higher levels and/or particularly active through a variety of mechanisms in tumors or other pathologies relative to normal tissues. In various tumors and other pathologies such receptors include receptors for epidermal growth factor, vascular endothelial growth factor, basic fibroblast growth factor, acidic fibroblast growth factor, nerve growth factor, platelet-derived growth factor, transforming growth factor-beta, insulin-like growth factor, hepatocyte growth factor, receptors for chemokines and lymphokines. Another group of suitable targets known in art are tumor specific antigens. The antigen binding moiety may be an antibody or fragment thereof perhaps derived from a monoclonal antibody which can be raised by methods known in the art, the antigen binding moiety may bind to surface antigenic determinants of the target cells.

Targets of specific embodiments of the present invention include receptors for epidermal growth factor expressed on the surface of tumor cells and receptors for vascular endothelial growth factor expressed on the surface of vascular endothelial cells, particularly on the surface of tumor and contiguous host vasculature, lymphatic endothelial cells, and some tumor cells.

Reference herein to a "gene" including an *subA, subB, BiP,* or genes encoding antisense, co-suppression, ribozyme, duplex RNA molecules or the like, is to be taken in its broadest context and includes a classical genomic gene having a transcribed region associated with regulatory regions such as promoters and transcription terminators-polyadenylation sequences. The transcribed region includes transcribed but not translated sequences (untranslated sequences, UTR) and optionally may include a protein coding region or introns, which are spliced out to form a mature RNA, or any combination of these. A "gene" includes forms obtained from cDNA, corresponding to the exons, and RNA genes such as those found on RNA genomes. The term "gene" is also used to describe synthetic or fusion molecules encoding all or part of a functional product.

When present in a cell, a "gene" directs the "expression" of a "biologically active" molecule or "gene product", which may be RNA or a polypeptide. This process is most commonly achieved by transcription to produce RNA and translation to produce protein. Such a product may be subsequently modified in the cell. RNA may be modified by, for example, polyadenylation, splicing, capping, dicing into 21-23 nucleotide fragments, or export from the nucleus or by covalent or noncovalent interactions with proteins. Proteins may be modified by, for example, phosphorylation, glycosylation or lipidation. All of these processes are encompassed by the term "expression of a gene" or the like as used herein.

### Transgenes

The expression and/or activity of *subA* or *subB* or other genes may be added by introducing one or more transgenes into an animal, preferably non-human animal. A "transgene" as referred to herein has the normal meaning in the art of biotechnology and includes a genetic sequence which has been produced or altered by recombinant DNA or RNA technology and which has been introduced into the organism or cell, of interest. The transgene typically includes an exogenous nucleic acid, which is not derived from said organism or cell. "Transgenic" refers to the organism or cell containing a transgene, or the genetic sequence that was introduced into the organism or cell or its progenitor. "Non-transgenic" refers to the absence of any transgene in the genome. A transgene is preferably integrated into the genome of the organism or cell, for stable inheritance.

Those skilled in the art will be aware that expression of a gene or a complementary sequence thereto in a cell requires the gene to be placed in operable connection with a promoter sequence. The choice of promoter for the present purpose may vary depending upon the level of expression required and/or the tissue, organ and species in which expression is to occur.

Placing a nucleic acid molecule under the regulatory control of a promoter sequence means positioning said molecule such that expression is controlled by the promoter sequence. A promoter is usually, but not necessarily, positioned upstream, or at the 5'-end, of the nucleic acid molecule it regulates. Furthermore, the regulatory elements comprising a promoter are usually positioned within 2 kb of the start site of transcription of the gene. In the construction of heterologous promoter/structural gene combinations, it is generally preferred to position the promoter at a distance from the gene transcription start site that is approximately the same as the distance between that promoter and the gene it controls in its natural setting (i.e., the gene from which the promoter is derived). As is known in the art, some variation in this distance can be accommodated without loss of promoter function. Similarly, the preferred positioning of a regulatory sequence element with respect to a heterologous gene to be placed under its control is defined by the positioning of the element in its natural setting (i.e., the gene from which it is derived). Again, as is known in the art, some variation in this distance can also occur.

Examples of promoters suitable for use in gene constructs of the present disclosure include promoters derived from the genes of viruses, yeast, moulds, bacteria, insects, birds, mammals and plants, preferably those capable of functioning in mammalian cells, more preferably for some embodiments of this disclosure those capable of being expressed in brain cells. The promoter may regulate expression constitutively, or differentially, with respect to the tissue in which expression occurs. Alternatively, expression may be differential with respect to the developmental stage at which expression occurs, or in response to external stimuli such as physiological stresses, or temperature.

### Genetic constructs/vectors

Disclosed are isolated nucleic acid molecules comprising RNA or DNA, preferably DNA. Also disclosed are genetic constructs comprising or encoding the isolated nucleic acid molecule, comprising one or more regulatory elements such as promoters, enhancers and transcription termination or polyadenylation sequences. Such elements are well known in the art. The genetic constructs may also comprise intron sequences that aid expression of the transgene in mammals. The term "intron" is used in its normal sense as meaning a genetic segment that is transcribed but does not encode protein and which is spliced out of an RNA before translation. Introns may be incorporated in a 5'-UTR or a coding region if the transgene encodes a translated product, or anywhere in the transcribed region if it does not.

Also disclosed are vectors, for example plasmid vectors, comprising the genetic constructs. The term "vector" includes an expression vector, being capable of *in vitro* or *in vivo* expression, and a transformation vector, capable of being transferred from one cell or organism to another. The vectors comprise sequences that provide for replication in cells, for example, in prokaryotic cells such as *E. coli.*

### Proncoters/termiators

In a disclosed embodiment, the transgene or other genetic construct includes a transcriptional initiation region (promoter) that may provide for regulated or constitutive expression in the endosperm of wheat. The promoter may be tissue specific, conferring expression selectively or exclusively in the endosperm. The promoter may be modulated by factors such as temperature, light or stress. Ordinarily, the promoter would be provided 5' of the gene to be expressed. The construct may also contain other elements that enhance transcription such as the nos 3' or the ocs 3' polyadenylation regions or transcription terminators. The regions of DNA illustrated will be incorporated into vectors containing suitable selectable marker gene sequences and other elements, or into vectors that are co-transformed with vectors containing these sequences.

As used herein, the term "isolated" is interpreted to mean altered "by the hand of man" from its natural state, i.e., if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein.

In the context of this disclosure, the term "purified", means that the specified nucleic acid molecule or polypeptide has been separated from other nucleic acid molecules or polypeptides, respectively, with which it is found in such a manner that it forms a substantial fraction of the total nucleic acids or polypeptides present in a preparation. Preferably, the specified molecule constitutes at least 1, 5, 10, 50, 75, 85, or 95 percent or more of the molecules of that type (nucleic acid or polypeptide) present in a preparation.

As used herein, the terms "altering", "increasing", "increased", "reducing", "reduced", "inhibited" or the like are considered relative terms, i.e. in comparison with the wild-type or unaltered state. The "level of a protein" refers to the amount of a particular protein, for example SubA, which may be measured by any means known in the art such as, for example, Western blot analysis or other immunological means. The "level of an enzyme activity" refers to the amount of a particular enzyme measured in an enzyme assay. It would be appreciated that the level of activity of an enzyme might be altered in a mutant if a more or less active protein is produced, but not the expression level (amount) of the protein itself. Conversely, the amount of protein might be altered but the activity (per unit protein) remain the same. Reductions in both amount and activity are also possible such as, for example, when the expression of a gene encoding the enzyme is reduced transcriptionally or post-transcriptionally. In certain embodiments, the reduction in the level of protein or activity is by at least 40% or by at least 60% compared to the level of protein or activity, or by at least 75%, at least 90% at least 95% or at least 99%. The term "wild-type" as used herein has its normal meaning in the field of genetics and includes genotypes which are not modified as taught herein.

Disclosed is a method of cleaving Immunoglubulin Binding Protein (BiP) including the step of contacting BiP with subunit A of subtilase cytotoxin (SubA) or an active variant thereof capable of specifically cleaving BiP between two adjacent hinge leucine residues.

The cleavage may be performed in a cell free system, alternatively however the cleavage may be in a cell that is part of a tissue whereby the SubA is provided in combined form with the SubB as a holotoxin, to allow for transport into the cell, and the method further comprises the step of monitoring the cell or tissue for changes induced by SubA.

Alternatively the cell is a recombinant cell comprising an exogenous polynucleotide encoding SubA or active variant thereof operably linked to an inducible promoter, the method of contacting including the step of inducing the promoter to cause expression of the SubA within the cell. The cells and/or cell lines are preferably tissue specific cell lines, such as, for example, a neuronal In cell line eg NF2C, a mammary cell line eg. B2LT1, a liver cell line, or a kidney cell line. Cell lines NF2C and B2LT1 have been deposited with the ECACC European Collection of Cell Cultures, CAMR (Center for Applied Microbiology and Research), Salisbury, Wiltshire, SP4 OJG, England, as Accession numbers 96092754 and 97032720 respectively.

Cleavage may be thus *in vitro* wherein purified SubA or active variant thereof or holotoxin is added to purified BiP or variant thereof. Alternative purified SubA or active variant may be added to a cell or tissue *in vitro* in this alternative approach it is preferred that the holotoxin is added, to assist with transport of the SubA catalytic subunit into the cell or alternatively the SubA or an active fragment thereof is part of a fusion protein or a protein conjugate, which comprises a receptor or an antigen binding moiety that can be used to target a surface of the target cell, in a particular specific embodiment this receptor binding moiety may be selected to induce internalisation of the SubA fragment. Alternatively purified SubA or active variant thereof or holotoxin comprising SubA or active variant thereof may be provided to specific tissue *in situ* in an animal, or to the whole animal.

Preferably the cleavage of BiP is monitored either directly by assaying for example for an increase in cleavage product or assaying for a decrease in uncleaved BiP. Alternatively assaying may be monitored indirectly for example for a product or a state that results from reduced BiP activity. Thus for example BiP cleavage may result in localised cell death, or simply in an increase in accumulation of misfolded proteins, or for a decrease in localised unfolded protein response (UPR) reaction especially when conditions of ER stress are applied.

Disclosed are also purified cleavage fragments of BiP or variant thereof resulting from cleavage by SubA of the subtilase toxin. Thus when derived from BiP these may comprise an approximately 44 kDa fragment and a 28 kDa fragment, comprising a separate ATPase domain and a separate protein binding domain of BiP.

Further disclosed is a method of reducing the level or activity of BiP protein in an animal cell by contacting the cell with a sublethal dose of SubA of the subtilase toxin or an active variant thereof capable of specifically cleaving BiP between the two adjacent hinge leucine residues. The cell may be part of a tissue, and some but not all of the cells of the tissue are contacted with SubA.

The animal may be a mammal, including a non-human mammal, or a human.

Preferably the assembled toxin is delivered, in the SubAB form so that toxin can readily be assimilated by the cell. Generally SubAB will kill cells very rapidly at high doses, but a sublethal dose may allow for the cells to survive for an extended period, thus for example 12 hours or more preferably 1, 2, 3, 5, or 10 days or more, to allow for the effects of the reduced level of BiP to be assessed. In a tissue culture setting it is probable that the cells will not proliferate once SubAB has contacted them, however the reduction in BiP may be sufficiently mild to allow for proliferation.

The sublethal level can be assessed by a titration experiment perhaps using trypan blue exclusion as a measure. Alternatively and perhaps more preferably a metabolic method may be used to assess viability.

It may be preferred to contact the BiP with a less active form of SubA, such as for example with a mutation at one or more of the three amino acids important for catalytic action, thus amino acid substitutions set out in WO2005061714 may be used for example Ser₂₇₂-Ala, Asp₅₂-Ala or His₈₉-Ala whereby BiP is not necessarily cleaved, and may bind the BiP. Alternatively an active variant of SubA may be used that is simply less effective, whereby perhaps more control can be achieved over the reduction in BiP level or activity.

Alternatively the cell into which it is introduced may be a recombinant cell comprising an exogenous polynucleotide encoding a resistant BiP being resistant to SubA cleavage, for example having an amino acid substitution at amino acid 416, or 417 or both. The expression of the resistant BiP may be at levels reduced relative to the endogenous BiP. This may be particularly applicable where SubA is provided as a *subA* gene on an exogenous polynucleotide.

### Cancers

BiP is a Ca 2+ binding molecular chaperone localized in the endoplasmic reticulum. Generally, the BiP protein has been implicated in the regulation of apoptosis and overexpression of BiP in tumor cells provides for several defence mechanisms. The anti-apoptotic function of BiP has been correlated with cancer progression and drug resistance. Recently, suppression of the BiP induction by antisense cDNA in murine fibrosarcoma cells was shown to increase apoptotic cell death and inhibit tumor formation in mice. Induction of BiP has also been correlated with the resistance to chemotherapeutic drugs such as etoposide, doxorubicin, camptothecin, vincristine, fudarabine, the retinoid analog, and a non-coxib analogue of celecoxib. Another protective mechanism associated with BiP overexpression in tumor cells may be based on the ability of BiP to slow cell growth and metabolism. Growth arrest could be an important cellular defence mechanism, as best exemplified by the DNA-damage response. In fact, it has been shown that induction of BiP in a human epidermoid carcinoma cell line results in a down-regulation of EGF-signaling pathway through a stable BiP-EGFR complex formation and subsequent growth arrest of cancer cells. These and other evidence indicate that induction of BiP is important for survival of cancer cells and development of their drug resistance.

Natural induction of BiP in multiple types of solid tumors can be attributed to glucose starvation resulting from poor perfusion within tumors as well as metabolic characteristics of cancer cells, which have much higher glucose utilization rates. BiP induction under pathological conditions may represent a major cellular protective mechanism for cells and it is proposed that reducing the amount of BiP in targeted cells can play a role in controlling cancer progression.

The invention provides a method of reducing the amount of BiP in a cancer cell, the cancer cell being part of or derived from a tumor. The method thus comprises delivering SubA or an active variant thereof to a tumor, preferably a solid tumor.

The solid tumor may be a cancer such as a carcinoma, melanoma, lymphoma, or sarcoma.

The SubA or an active variant thereof may be introduced either systemically or intratumorally, for example, by injection perhaps together with an agent to localise subA or an active variant thereof for example a poly(phosphoester) biodegradable polymer in a method described in US Patent 6537585 to Dang and Garver. "Intratumoral" administration means implanting a reservoir of a therapeutic agent(s) inside a tumor. Intratumoral administrations is advantageous for tumor treatment because the outer cell layers of tumors are often composed of a high percentage of necrotic cells and/or connective and support tissue which slow and/or impede the extra-tumoral vascular or parenteral delivery of therapeutic agents to the actively growing cancer cells at the center of solid tumors. It will be understood that this form administration is advantageous because SubA or an active variant thereof have a substantial size compared to many antineoplastic compounds. It will also be understood that it is preferred that the holotoxin be administered, to facilitate transport of the effector subA molecule into the tumor cells.

In one form SubA or an active variant thereof is administered together with one or more other antineoplastic compounds used in the treatment of tumors, particularly solid tumors.

Alternatively the SubA or active variant thereof delivered together with an ER (Endoplasmic Reticulum) stress inducer.

It is to be understood that various co-adminstration regimes may be used to deliver the Sub A together with the further therapeutic agent, being either an antineoplastic compounds or the ER stress inducer. Such coadminstration may include, where the further therapeutic agent is a substance, delivery in the same packaging, and thus at the same time, alternatively delivery in a different package or through a different route albeit co-extensive. Alternatively delivery can be temporally spaced apart. Similarly where the further therapeutic agent is not a substance, for example radiation therapy, this may be delivered at essentially the same time or temporally spaced apart. The term coadministration is thus not restricted to delivery at the same time provided that delivery of the SubA and the further therapeutic agent provide a cumulative effect.

The present invention further provides a pharmaceutical composition for treating cancer in an animal comprising a therapeutically effective amount of SubA or active variant thereof and a pharmaceutically acceptable carrier. This can take a variety of forms adapted to the chosen route of administration as discussed above.

Preferred embodiments of the invention comprise physiologically acceptable compositions and methods of treating cancer in a patient (such as prostate cancer, skin cancer/melanoma, pancreatic carcinoma, colon cancer, melanoma, ovarian cancer, liver cancer, small cell lung carcinoma, non-small cell lung carcinoma, cervical cancer, breast cancer, bladder cancer, brain cancer, neuroblastoma/glioblastoma, leukemia, lymphoma, head and neck cancer, kidney cancer, myeloma and ovarian cancer) characterized by proliferation of neoplastic cells and proliferation of endothelial cells for development of tumor vasculature, which comprises administering to the patient an amount of SubA or an active variant thereof, effective to: (a) selectively induce apoptosis and/or necrosis in such neoplastic cells or endothelial cells relevant to development of tumor vasculature and thereby inhibit their proliferation; (b) inhibit cell growth and proliferation of the neoplastic cells or endothelial cells relevant to development of tumor vasculature; (c) inhibit invasion of the neoplastic cells; (d) inhibit metastasis of the neoplastic cells; (e) kill neoplastic cells; (f) preferentially inhibit cell growth and proliferation of the neoplastic cells or endothelial cells relevant to development of tumor vasculature; or (g) preferentially kill neoplastic cells or endothelial cells relevant to development of tumor vasculature. SubA or an active variant thereof can be used in combination with one or more of various anticancer treatments known as cancer chemotherapeutic agents and/or radiation therapy and/or an ER stress inducer. The active ingredient compound of the invention which can produce an excellent anticancer effect can thus markedly promote the effect of the other anticancer agent or agents used in combination, to produce a synergistic effect. Therefore, even when the partner anticancer agent or agents are used in doses much smaller than the usual doses, a satisfactory anticancer effect can be obtained, whereby the adverse effects of the partner anticancer agent or agents can be minimized. As such chemotherapeutic agents include but are not limited to, for example, 5-fluorouracil (5-FU; Kyowa Hakko Kogyo), mitomycin C (Kyowa Hakko Kogyo), futraful (FT-207; Taiho Pharmaceutical), endoxan (Shionogi & Co.), toyomycin (Takeda Chemical Industries), etoposide, doxorubicin, camptothecin, vincristine, fudarabine, the retinoid analog, and non-coxib analogue of celecoxib. In addition, the apoptosis regulating composition of the present invention may be administered with a vitamin D derivative to further enhance its cytotoxic characteristics (U.S. Pat. No. 6,087,350). The anti-cancer agents of the present invention may be combined with an anti-oestrogen compound such as tamoxifen or anti-progesterone such as onapristone (see, EP 616812) in dosages known for such molecules. In addition, the apoptosis regulating composition of the present invention may be administered with ER stress inducing compound that can be selected from a group that includes, but not limited to, inhibitors of calcium dependent endoplasmic reticulum ATPase, inhibitors of proteasome, inhibitors of protein glycosylation, and compounds capable of inducing glucose or oxygen deprivation by targeting tumor angiogenesis and tumor vasculature.

The pharmaceutically and physiologically acceptable compositions utilized in this invention may be administered by any number of routes including, but not limited to, parenteral, subcutaneous, intracranial, intraorbital, intracapsular, intraspinal, intracisternal, intrapulmonary (inhaled), oral, intravenous, intramuscular, intra-arterial, intramedullary, intrathecal, intraventricular, transdermal, subcutaneous, intraperitoneal, intranasal, enteral, topical, sublingual, or rectal means. In addition to the active ingredients, these pharmaceutically and physiologically acceptable compositions may contain suitable physiologically acceptable carriers comprising excipients and auxiliaries, which facilitate processing of the active compounds into preparations, which can be used pharmaceutically. Further details on techniques for formulation and administration may be found in the latest edition of Remington's Pharmaceutical Sciences (Mack Publishing Co. Easton, Pa.). Pharmaceutically and physiologically acceptable compositions for oral administration can be formulated using physiologically acceptable carriers well known in the art in dosages suitable for oral administration. Such carriers enable the pharmaceutically and physiologically acceptable compositions to be formulated as tablets, pills, dragees, capsules, liquids, gels, syrups, slurries, suspensions, and the like, for ingestion by the patient. Pharmaceutical preparations for oral use can be obtained through a combination of active compounds with solid excipient, suiting mixture is optionally grinding, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titaniumdioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations, which can be used orally, include push-fit capsules made of gelatin, as well as soft, sealed capsules made of gelatin and a coating, such as glycerol or sorbitol. Push-fit capsules can contain active ingredients mixed with filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers. Pharmaceutical formulations suitable for parenteral administration may be formulated in aqueous solutions, preferably in physiologically compatible buffers such as Hanks solution, Ringer's solution, or physiologically buffered saline. Aqueous injection suspensions may contain substances, which increase the viscosity of the suspension, such as sodium carboxymethylcellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension may also contain suitable stabilizers or agents, which increase the solubility of the compounds to allow for the preparation of highly, concentrated solutions. For topical or nasal administration, penetrants appropriate to the particular barrier to be permeated are used in the formulation. Such penetrants are generally known in the art. The pharmaceutically and physiologically acceptable compositions of the present invention may be manufactured in a manner that is known in the art, e.g., by means of conventional mixing, dissolving, granulating, dragee-making, levigating, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation may be a lyophilized powder which may contain any or all of the following: 1 50 mM histidine, 0.1% 2% sucrose, and 2 7% mannitol, at a pH range of 4.5 to 5.5, that is combined with buffer prior to use. After pharmaceutically and physiologically acceptable compositions have been prepared, they can be placed in an appropriate container and labelled for treatment of an indicated condition. For administration of sub A or active variant thereof, such labeling would include amount, frequency, and method of administration. Pharmaceutically and physiologically acceptable compositions suitable for use in the invention include compositions wherein the active ingredients are contained in an effective amount to achieve the intended purpose. The determination of an effective dose is well within the capability of those skilled in the art. For any compound, the therapeutically effective dose can be estimated initially either in cell culture assays, e.g., of neoplastic cells, or in animal models, usually mice, rabbits, dogs, or pigs. The animal model may also be used to determine the appropriate concentration range and route of administration. Such information can then be used to determine useful doses and routes for administration in humans. Those of ordinary skill in the art are well able to extrapolate from one model (be it an in vitro or an in vivo model). A therapeutically effective dose refers to that amount of active ingredient, for example sub A or active variant thereof, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Pharmaceutically and physiologically acceptable compositions, which exhibit large therapeutic indices, are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage form employed, sensitivity of the patient, and the route of administration. The practitioner, in light of factors related to the subject that requires treatment, will determine the exact dosage. Dosage and administration are adjusted to provide sufficient levels of the active moiety or to maintain the desired effect. Factors, which may be taken into account, include the severity of the disease state, general health of the subject, age, weight, and gender of the subject, diet, time and frequency of administration, drug combination(s), reaction sensitivities, and tolerance/response to therapy. Long-acting pharmaceutically and physiologically acceptable compositions maybe administered every 3 to 4 days, every week, or once every two weeks depending on half-life and clearance rate of the particular formulation. Normal dosage amounts may vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. For the prevention or treatment of disease, the appropriate dosage of an anti-tumor agent herein will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" in Toxicokinetics and New Drug Development, Yacobi et al., eds., Pergamon Press, New York 1989, pp. 42 96. For example, depending on the type and severity of the disease, about 1 g/kg to 15 mg/kg (e.g., 0.1 20 mg/kg) of an antitumor agent is an initial candidate dosage for administration to the patient, whether, for example, by one or more separate administrations, or by continuous infusion. A typical daily dosage might range from about 1 g/kg to 100 g/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue. Therapies may be designed to utilize sub A cytotoxic properties. In particular, therapies to enhance subA expression or administration of said polypeptides are useful in promoting inhibition or death of cancerous cells.

Another therapeutic approach within the invention involves administration of SubA therapeutic compositions, either directly to the site of a desired target cell or tissue (for example, by injection) or to a site where the composition will be further directed to the target cell or tissue, or systemically (for example, by any conventional recombinant protein administration technique). The dosage of SubA depends on a number of factors, including the size and health of the individual patient, but, generally, between 0.1 mg and 100 mg inclusive is administered per day to an adult in any physiologically acceptable formulation.

### Conformational diseases

Protein misfolding is implicated in the pathogenesis of many genetic disease. Mostly these disease-causing mutations affect the ability of the protein to fold into a correct conformation, and often give rise to premature degradation or aggregation of the mutant proteins. Disease caused by this kind of molecular pathological mechanism are termed "conformational diseases".

The present invention has application to conformational diseases because BiP is a principal component in disposing of misfolded proteins or assisting with their correction. Cleaving BiP to stop or reduce BiP level and/or activity in a cell or perhaps a cell free system provides a means for screening for a substance that may correct or otherwise influence the misfolding.

Disclosed is an *in vitro* method of determining the effect of a substance, or physical condition or characteristics that are indicative of a conformational disease in eukaryotic cells, said method comprising (1) exposing said cells to a sublethal dose of SubA toxin or active variant thereof that cleaves BiP at the two adjacent hinge leucine residues (2) maintaining said cells for a time that is sufficient to reduce the level of BiP in said cells, (3) adding said substance, or apply the physical condition before, during and/or after said exposing or said maintaining and (4) determining whether the presence of said substance has an effect on the induction of said one or more characteristics.

The conformational disease may be selected from the group consisting of Alzheimer's disease, Parkinson's disease, Huntington's disease, Cataracts, Ischemia, Epileptic seizures, Prion protein disease, Amytrophic lateral sclerosis, ageing, Sjorgen's syndrome, Inclusion body myositis, Bipolar disorder, retinopathy, Cataracts and Cystic Fibrosis.

In an alternative form the conformational disease is a neurodegenerative disease and thus may be Alzheimer's disease progressive supranuclear palsy, postencephaltic frontotemporal dementias, frontotemporal dementia and Parkinsons disease, Huntingon's disease, Ischemia, Pick's disease, Progressive supranuclear palsy pathology, Senile dementia, Stroke, Amylotrophic lateral sclerosis and others especially where neurofibrillary tangles are a feature.

Thus the disclosure provides a method of determining the effect of a substance or physical condition on characteristics that are indicative of a Neurodegenerative disease in brain or neural cells, said method comprising (1) exposing said cells to a sublethal dose of SubA toxin or active variant thereof that cleaves BiP between the two adjacent leucine hinge residues (2) maintaining said cells for a time that is sufficient to reduce the level of BiP in said cell, (3) adding said substance or physical condition before, during and/or after said exposing or said maintaining and (4) determining whether the presence of said substance has an effect on the induction of said one or more characteristics.

Age-related neurodegenerative diseases and neurodegenerative diseases are characterized by many of the same properties including, e.g., an increased amount of neurofibrillary tangles and/or lysosomes, the appearance of basophilic granules in the mossy fibre terminal zone, the presence of secondary lysosomes with lipofuscin, amyloid deposition, amyloid plaques, neuritic plaques, synaptic loss, neuritic degeneration, neuronal death, increased glial elements (astrocytes, microglia), the fragmentation of the amyloid precursor protein, increased levels of Cathepsin D, inflammation, etc. Brain aging refers to a condition in which brain cells mimic the biochemistry or physiology of brain cells taken from a mature or elderly animal, especially human. Brain aging is manifested by significant changes in lysosomal functions and chemistry, e.g., the proliferation of secondary lysosomes filled with lipofuscin, decreases in cathepsin L activities and increases in the levels of cathepsin D. Additional characteristic features of human brain aging, as is found in neurodegenerative diseases, and especially Alzheimer's disease, include depletion of synaptic proteins, meganeurite formation, induction of early-stage tangles, and accompanying tau proteolysis. Any one or more of these and other characteristics may be monitored in assessing the effect of a substance on Alzheimer's disease or any one or more the neurodegenerative disease referred to herein.

The characteristics may be selected from the group consisting of a) the formation of neurofibrillary tangles, b) the hyperphosphoryation of tau c) the fragmentation of tau d) the production and/or release of brain produced cytokines TGF-beta, IL-1b or TNF e) a microglia reaction or microglial activation f) indications of brain inflammatory reactions g) conversion of p35 to p25 h) changes in the level and/or activity of cycling dependent protein kinase 5 (cdk5), i) changes in the level and/or activity of mitogen activated proteinkinase (MAPK), j) changes in the level and/or activity of phosphorylated (activated) pancreatic ER kinase (p-PERK) wherein said effect on said induction of any or all of said characteristics in a) to i) is indicative of the appearance or disappearance respectively of said characteristics of Alzheimer's Disease.

Any suitable source of brain cells can be used as they relate to neurodegenerative diseases. Typically, brain cells are derived from a mammal, such as a mouse, rat, guinea pig, rabbit, or the like. Primary cell cultures, including human primary cell cultures, can also be used in the methods of the disclosure. Brain cells can be derived from a normal animal or other suitable animals. A preferred embodiment includes *in vivo* brain cells. The method may be an *in vitro* method, and the brain cell may be a rodent brain cell, particularly that of a rat or a mouse.

### Alzheimer's

There is evidence that ER dysfunction plays a major role in the development of neuronal cell injury in Alzheimer's disease (Katayama et al., (1999) Nature Cell Biol 1;479 485). Mutations in the presenilin-1 gene, causing early onset familial Alzheimer's diseases (FAD), down regulate the signalling pathway of UPR. Expression of mutant presenilins induces a decrease in BiP protein levels and an increase in the sensitivity of cells to conditions associated with ER stress (Katayama *et al.,* 1999). This pathological process was completely blocked in cells over-expressing BiP (Katayama et al., 1999). BiP expression also was increased in successfully surviving neurons (Hamos et al., 1991; Neurology 41,345-350.) It has been suggested that mutations in the presenilin-1 gene disrupt the action of IRE 1, and ER-membrane protein, which plays a central role in the signalling pathway activated under conditions of ER dysfunction. Phosphorylation of Ere1p is responsible for the activation of the expression of genes coding for ER-resident stress proteins. The authors did indeed find a marked decrease of BiP protein levels in the brains of FAD patients (Katayama et al. 1999). Furthermore, it has been shown that activation of UPR leads to proteolytic cleavage of IRE1, and accumulation of IRE1 fragments in the nucleus, and that this process is disturbed in presenilin-1 knock-out cells. The role played by IRE1 in the signalling pathway of UPR is indicated by the observation that under conditions of ER stress the apoptotic process is markedly increased in cells expressing a dominant-negative form of IRE1. However, the precise role or presenilins in the induction of UPR, and the consequence of presenilin mutations on UPR are still a matter of debate.

The exact molecular mechanisms underlying the development of neuronal cell injury in sporadic Alzheimer's disease (SAD) are less well understood. A novel presenilin-2 splice variant has been found in the brains of SAD patients. This new splice variant is also produced under hypoxic conditions, and cells expressing this splice variant are more vulnerable to stress conditions such as hypoxia, tunicamycin and calcium ionophore exposure. Recently this presenilin-2 splice variant has been shown to cause significant increases in Ab40 and Ab42 production, and also to interfere with the signalling pathway of UPR by blocking IRE1p phosphorylation, and to increase the sensitivity of cells to conditions associated with ER stress.

It is thus thought that the Unfolded Protein Response (UPR) as a reaction to endoplasmic reticulum (ER) stress plays a role in Alzheimer disease pathogenesis. It has been suggested that experimental manipulation of BiP expression might allow the development of therapeutic strategies for Alzheimer disease (Katayama et al., Ann NY Acad Sci 2002; 977:349-55).

Thus the disclosure provides a method of determining the effect of a substance or physical condition on characteristics that are indicative of Alzheimers disease and in particular the cells tested may be brain cells.

Although human tissue or tissue lines may also be used where it is available.

### Parkinson's

Parkinson's disease is an age-related disorder characterised by a progressive degeneration of dopaminergic neurons in the substantia nigra and showing a corresponding motor deficit. There is increasing evidence that shows that besides oxidative stress and mitochondrial dysfunction, protein folding defects are also key elements of Parkinson's disease etiology. Similar to Alzheimer's disease, glial and astroglial cells of Parkinson's disease victims showed the expression of αB-crystallin and similarly in neurofibrillary tangles aggregated proteins in Lewy bodies had a large content of various heat-shock proteins. Dietary restriction-induced expression of Hsp70 and BiP parallel with a protection in Parkinson's disease model. Interestingly, parkin, the protein whose mutation causes the autosomal recessive juvenile parkinsonism was identified as an ubiquitin-ligase playing a key role in the degradation of ER misfolded proteins, such as a G-protein coupled membrane receptor called Pael and synphilin, and α-synuclein interacting protein.

Thus the disclosure provides a method of determining the effect of a substance or physical condition on characteristics that are indicative of Parkinson's disease and in particular the cells tested may be brain cells, more preferably a hypothalamus derived brain cell.

The character may include checking for abnormal Parkin.

### Prion protein disease

"Prions are proteins, which have a structure trapped in a high-energy state containing α-helices. These "normal" prions are present in the brain, and cannot form large aggregates and are sensitive to proteolysis. If their conformational switches to the low-energy state, characterised by β-sheet formation, they become resistant to proteolysis and prone to excessive aggregation. The *in vivo* functions of prions are not entirely clear although it is thought that they play a role in signalling, in copper metabolism, in redox regulation and in the proteins of neurons. Prion aggregation, which is a cause of Creutzfeld-Jakob disease, involves many chaperons. Chaperones try to block the contact surfaces of "transformed" prion molecules. Many chaperones, such as Hsp60, Hsp70 and its co-chaperone, Hsp40 act to prevent prion aggregation. Aggregated prions are also subject to proteolytic attack including labelling for proteasomal degradation, and ubiquitinylation. Since prions are also extracellular proteins attached to the plasma membrane, the fight against their transformed (mutant) form begins in the ER. BiP binds to these prions and mediates their degradation by proteasome. A diminution of BiP levels in the cells will accordingly have an interesting effect on the capacity for prions to aggregate.

Thus the disclosure provides a method of determining the effect of a substance or physical condition on characteristics that are indicative of a Prion protein disease and in particular the cells tested may be brain cells.

The prion protein disease in one form may be Creutzfeld Jacob disease.

### Huntington's and other polyglutamine diseases

Polyglutamine repeats make proteins vulnerable to aggregation. Diseases, such as Huntington's disease, Kennedy spinal bulbar muscular atrophy, spinocerebral ataxia, Machado-Joseph disease, and others all develop due to an expansion to polyglutamine segments in the respective proteins. Chaperones co-localize with the aggregates of these polyglutamine-containing proteins and increases chaperone levels, such as that of Hsp40, Hsp60, Hsp70, Hsc70, Hsp100 inhibit polyglutamine-containing protein aggregation and slow down the progress of disease.

Thus the disclosure provides a method of determining the effect of a substance or physical condition on characteristics that are indicative of a polyglutamine disease and in particular the cells tested may be brain cells.

The characteristics may be selected from the group consisting of polyglutamine protein aggregation and/or one or more of those characteristics indicated for Alzheimer's disease above.

The prion protein disease in one form may be selected from the group consisting of Huntington's disease, Kennedy spinal bulbar muscular atrophy, spinocerebral ataxia, Machado-Joseph disease.

### Sjorgen's Syndrome

There is evidence supporting the hypothesis that the ER chaperone BiP is a potential candidate in the initiation of an autoimmune response against Ro52, by binding to a BiP binding motif in the COOH-terminal region of Ro52, and thereby play a role in this common systemic autoimmune disease. (See Gordon et al Autoimmunity 2001 34(2) Thus the disclosure provides a method of determining the effect of a substance or physical condition on characteristics that are indicative of Sjorgen's Syndrome and in particular the cells tested may be brain cells.

### Amytrophic lateral sclerosis

Amyotrophic lateral sclerosis (ALS) is a devastating neurodegenerative disease that leads to paralysis and death within several years after onset due to the loss of motor neurons. Approximately 10-15% of ALS cases are familial, and the disease in approximately 15% of these patients is caused by dominantly inherited mutations in the copper- and zinc-dependent superoxide dismutase (SOD1) gene.

Data indicating the possibility that BiP binds to and coaggregates with mSOD1 to form certain abnormal structures in mSOD1 Tg mice has been obtained. The results suggest that dysfunction of BiP and subsequent derangement of the system responding to unfolded proteins may be involved in the pathogenesis of familial amyotrophic lateral sclerosis caused by a mutation of the human SOD1 gene. Wate et al (2005) Acta Neuropathol (2005) 110: 557-562.

Thus the disclosure provides a method of determining the effect of a substance or physical condition on characteristics that are indicative of Amytrophic lateral sclerosis and in particular the cells tested may be brain cells.

The characteristics may be selected from the group consisting of aggregated SOD1 with or without BiP and one or more of those characteristics indicated for Alzheimer's disease above.

### Seizures including Epileptic seizures, acute neuronal trauma, and bipolar disorders

BiP probably plays a role in the action of the drug VPA. The mood-stabilizing drug sodium valproate (VPA) is a branched-chain saturated fatty acid (2-propylpentanoic acid) that possesses anticonvulsant effects in the treatment of epilepsy. Evidence has established that VPA has a mood-stabilizing effect; this drug is quickly becoming a first line treatment for bipolar disorder (BD) because it has a broad spectrum of efficacy in patients with this illness. Chronic VPA treatment leads to an increased BiP mRNA expression (Wang et al. 1999, Molecular Pharmacology, 55:521-527) Increased BiP, but not HSP70, expression after VPA treatment suggests that this drug may specifically regulate protein processing linked to BiP induction. These results strongly suggest that specific genes, including the molecular chaperone BiP, are regulated by VPA treatment. Furthermore, BiP mRNA expression was also increased by treatment with the closely related mood-stabilizing anticonvulsant carbamazepine, but not lithium chloride. There is increasing interest in the role of BiP in CNS disease and injury. BiP expression is induced after CNS injuries such as seizures, global ischemia, and acute trauma, and in neurodegenerative diseases like Alzheimer's. BiP induction by chronic VPA treatment may play a neuroprotective role by clearing malfolded proteins.

Thus the disclosure provides a method of determining the effect of a substance or physical condition on characteristics that are indicative of seizures including epileptic seizures, acute neuronal trauma, and bipolar disorders and in particular the cells tested may be brain cells.

### Cataracts

The incidence of age-related cataracts increases with age and reaches 88% for people older than 75 years. One of the schemes proposed for the formation of cataracts in humans is the aggregation of crystallin protein into particles larger than 5x 10⁷ Da that occur in a highly oxidized environment, and these particles scatter light because of their high refractive index.

Cataract is a multifactorial disease, and a large variety of stressors induce cataracts. Many cataractogenic stressors and endoplasmic reticulum (ER) stressors induce the unfolded protein response (UPR) in various cell types. The UPR is known to produce reactive oxygen species (ROS) prior to the inducement of apoptosis. ER stressors are found induce the UPR in lens epithelial cells (LECs) or whole rat lenses and that higher levels of ER stressors activated Bip/GRP78, ATF4, and caspase-12. In addition, ROS are produced, free glutathione was decreased, and apoptosis was induced. LECs in the mitotic zone were the most susceptible to the UPR while the central LECs were the most resistant. The UPR induced the production of ROS in the ER and probably in the mitochondria. The detectable ROS production in cultured lenses is limited to the epithelial cells. These findings indicate that ER stressors induce the UPR in LECs with and without the induction of apoptosis, so that the UPR is probably one of the initiating factors of many types of cataracts. (Ikesuga et al., 2006, Experimental Eye Research 83; 508-516). BiP thus has a direct role in the formation of cataracts.

Thus the disclosure provides a method of determining the effect of a substance or physical condition on characteristics that are indicative of cataracts in eukaryotic cells, said method comprising (1) exposing said eye epithelial cell to a sublethal dose of SubA toxin or active variant thereof that cleaves BiP between the two adjacent hinge leucine residues (2) maintaining said cells for a time that is sufficient to reduce the level of BiP in said cell, (3) adding said substance before, during and/or after said exposing or said maintaining and (4) determining whether the presence of said substance has an effect on the induction of said one or more characteristics.

The characteristics may be selected from the group consisting of the presence of ROS, accumulation of crystallin protein, or UPR associated proteins.

Preferably the cells are eye epithelial cells.

### Familial hypercholesterolemia

Low density lipoprotein (LDL) receptor is a transmembrane glycoprotein that binds and internalizes circulating particles of LDL by receptor- mediated endocytosis. Mutations in the LDL receptor gene cause familial hypercholesterolemia (FH), which is an autosomal dominant inherited disorder of lipoprotein metabolism. Heterozygous FH is a common disorder with an estimated frequency of about 1 in 500. About 50% of the characterized mutations result in LDL receptor proteins that are retained in the endoplasmic reticulum (ER). Since the existence of an ER quality control system ensures that only correctly folded, newly synthesized proteins are transported to the plasma membrane or secreted, it is likely that protein misfolding contributes to the pathogenesis of FH. BiP interacts transiently with the wild type LDL receptor and displays prolonged interaction with two mutant LDL receptors. This indicates that misfolding of the two mutant LDL receptors causes retention in the ER and that BiP is involved in the specific retention of the mutant receptors in the ER. Accordingly, protein misfolding contributes to the pathogenesis of familial hypercholesterolemia. These results suggest that BiP is a potential key factor in the ER quality control of the newly synthesized LDL receptor." (Jorgensen 2000, JBC 275(43) 33861 - 33868).

Thus the disclosure provides a method of determining the effect of a substance on characteristics that are indicative of familial hypercholesterolaemia in an eukaryotic cell, said method comprising (1) exposing said eukaryotic cell to a sublethal dose of SubA toxin or active variant thereof that cleaves BiP between the two adjacent hinge leucine residues (2) maintaining said cells for a time that is sufficient to reduce the level of BiP in said cell, (3) adding said substance before, during and/or after said exposing or said maintaining and (4) determining whether the presence of said substance has an effect on the induction of said one or more characteristics.

The characteristics may be selected from the group consisting of accumulation of LDL receptor.

### ER stress

A central role is played by the chaperone BiP, as indicated by observations that overexpression of BiP protects cells from death associated with ER stress whereas down-regulation of BiP increases the sensitivity of cells to ER stress.

The disclosure provides an *in vitro* method of determining the effect of a substance on characteristics that are indicative of an Endoplasmic Reticular stress in eukaryotic cells, said method comprising (1) exposing said cells to a sublethal dose of SubA toxin or active variant that cleaves between the two adjacent hinge leucine residues (2) maintaining said cells for a time that is sufficient to reduce the level of BiP in said cell, (3) adding said substance before, during and/or after said exposing or said maintaining and (4) determining whether the presence of said substance has an effect on the induction of said one or more characteristics.

The characteristic may be selected from a modulation of levels of proteins associated with UPR EOR or other ER stress related factors.

Candidate substances can be virtually any agent, including without limitation metals, peptides, proteins, lipids, polysaccharides, small organic molecules, nucleotides (including non-naturally occurring ones) and combinations thereof. Small organic molecules have a molecular weight of more than 50 yet less than about 2,500 daltons, and most preferably less than about 400 daltons. Complex mixtures of substances such as natural product extracts, which may include more than one candidate, can also be tested, and the component that effects the change can be purified from the mixture in a subsequent step.

Candidate substances may be derived from large libraries of synthetic or natural compounds. For example, synthetic compound libraries are commercially available from Maybridge Chemical Co. (Trevillet, Cornwall, UK), Comgenex (Princeton, N.J.), Brandon Associates (Merrimack, N.H.), and Microsource (New Milford, Conn.). A rare chemical library is available from Aldrich (Milwaukee, Wis.). Alternatively, libraries of natural compounds in the form of bacterial, fungal, plant and animal extracts are available from Pan Labs (Bothell, Wash.) or MycoSearch (NC), or are readily producible. Additionally, natural and synthetically produced libraries and compounds are readily modified through conventional chemical, physical, and biochemical means. For example, the compounds may be modified to enhance efficacy, stability, pharmaceutical compatibility, and the like. For example, once a peptide ligand has been identified using the present disclosure, it may be modified in a variety of ways to enhance its stability, such as using an unnatural amino acid, such as a D-amino acid, particularly D-alanine, or by functionalizing the amino or carboxyl terminus, e.g., for the amino group, acylation or alkylation, and for the carboxyl group, esterification or amidification, or through constraint of the peptide chain in a cyclic form, or through other strategies well known to those skilled in the art.

Physical condition might be temperature, electromagnetic radiation, influence of a magnetic field, gamma rays.

The disclosure also provides a eukaryotic cell with an inducible *subA* gene, the induction of the *subA* gene preferably leading to sublethal expression of SubA or active variant thereof. The said cell is preferably a cultured cell line that is maintained *in vitro.*

The inducible *subA* gene may be incorporated into a chromosome of the cell, so that it replicates together with the normal cellular replication processes.

Alternatively the inducible *subA* gene may be carried on a self replicating polynucleotide to be separately replicated.

The eukaryotic cell may be selected from the group comprising brain or other neural cell, cells related to the eye, muscle cells including skeletal muscle.

The disclosure further provides a transgenic non-human animal, preferably a rodent, more preferably a mouse comprising the cell as disclosed herein. Preferably the *subA* gene is operably linked to a promoter that can be controlled to express a sublethal amount of SubA or an active variant thereof.

Preferably the promoter is regulated to be tissue specific. Tissue specific effects can be achieved, for example, in the case of virus mediated transport by using viral vectors that are tissue specific, or by the use of promoters that are tissue specific. For instance, any tissue-specific promoter may be used to achieve specific expression, for example albumin promoters (liver specific; Pinkert et al., 1987 Genes Dev. 1:268 277), lymphoid specific promoters (Calame et al., 1988 Adv. Immunol. 43:235 275), promoters of T-cell receptors (Winoto et al., 1989 EMBO J. 8:729 733) and immunoglobulins (Banerji et al., 1983 Cell 33:729 740; Queen and Baltimore 1983 Cell 33:741 748), neuron-specific promoters (e.g. the neurofilament promoter; Byrne et al., 1989 Proc. Natl. Acad. Sci. USA 86:5473 5477), pancreas-specific promoters (Edlunch et al., 1985 Science 230:912 916) or mammary gland-specific promoters (milk whey promoter, U.S. Pat. No. 4,873,316 and European Application Publication No. 264, 166). Developmentally-regulated promoters can also be used, such as the murine homeobox promoters (Kessel et al., 1990 Science 249:374 379) or the alpha-fetoprotein promoter (Campes et al., 1989 Genes Dev. 3:537 546).

A non-human "transgenic" animal can be produced by cross-breeding two chimeric animals which include exogenous genetic material within cells used in reproduction. Twenty-five percent of the resulting offspring will be transgenic, i.e., animals that include the exogenous genetic material within all of their cells in both alleles. Fifty percent of the resulting animals will include the exogenous genetic material within one allele and twenty five percent will include no exogenous genetic material.

Various methods to make the transgenic animals of the subject disclosure can be employed. Generally speaking, three such methods may be employed. In one such method, an embryo at the pronuclear stage (a "one cell embryo") is harvested from a female and the transgene is micro injected into the embryo, in which case the transgene will be chromosomally integrated into both the germ cells and somatic cells of the resulting mature animal.

In another method non-human embryonic stem cells are isolated and the transgene incorporated therein by electroporation, plasmid transfection or microinjection, followed by reintroduction of the stem cells into the embryo where they colonize and contribute to the germ line. Methods for microinjection of mammalian species is described in U.S. Pat. No. 4,873,191.

In yet another such non-human embryonic cells are infected with a retrovirus containing the transgene whereby the germ cells of the embryo have the transgene chromosomally integrated therein. When the animals to be made transgenic are avian, because avian fertilized ova generally go through cell division for the first twenty hours in the oviduct, microinjection into the pronucleus of the fertilized egg is problematic due to the inaccessibility of the pronucleus. When the animals to be made transgenic are bovine or porcine, microinjection can be hampered by the opacity of the ova thereby making the nuclei difficult to identify by traditional differential interference-contrast microscopy. To overcome this problem, the ova can first be centrifuged to segregate the pronuclei for better visualization.

The "non-human animals" of the disclosure include murine, bovine, porcine, ovine, piscine and avian animals.

The disclosure also provides a SubA-resistant BiP. In one form one or both of the two adjacent hinge leucine residues are altered. Alteration may provide a substitution perhaps a conservative substitution. Other aspects of the disclosure include a cell comprising the SubA-resistant BiP, or an animal comprising the cell above, or a polynucleotide encoding a SubA-resistant BiP.

### EXAMPLE 1

STEC are an important cause of gastrointestinal disease in humans, particularly since these infections may result in life-threatening sequelae such as the haemolytic uraemic syndrome (HUS). Much of the pathology associated with STEC disease is thought to be due to systemic effects of Stx, but other factors are clearly important, and STEC strains differ markedly in their virulence for humans³. Subtilase cytotoxin (SubAB) was discovered in a highly virulent O113:H21 STEC strain, which was responsible for an outbreak of HUS in South Australia in 1998, and has since been detected in several other STEC serotypes^{2,4}. SubAB is a potential bioterrorism agent, as it is more toxic for Vero (African green monkey kidney) cells than Shiga toxin, and is lethal for mice, resulting in extensive microvascular damage, thrombosis and necrosis in multiple organs, including the brain, kidneys and liver². It was named "Subtilase cytotoxin", because its 35-kDa A subunit (SubA) shares sequence homology to a subtilase-like serine protease of *Bacillus anthracis²,* although the sequence identity was low (26%). Subtilases are characterised by a conserved catalytic triad (Asp, His, Ser) and are found in a wide variety of microorganisms. However, none had previously been shown to have cytotoxic properties⁵. Mutagenesis of the critical Ser₂₇₂ residue in SubA to Ala abolished *in vitro* and *in vivo* toxicity. Thus, serine protease activity is central to the mechanism of action of the holotoxin².

### METHODS

### Purified proteins

SubAB, its non-toxic derivative SubA_{A272}B, and SubA were purified by Ni-NTA chromatography as described previously^{2,18}. Purified bovine BiP was obtained from Sigma (St. Louis, MO).

### Cell Culture and Cytotoxicity Assays

Vero cells were routinely grown in DMEM medium. For cytotoxicity assays, confluent monolayers in 96-well flat-bottom trays were washed with PBS, treated with 50 µl of toxin (serially diluted in DMEM without FCS), and incubated at 37°C for 30 min. 150 µl of medium supplemented with 2% FCS was added per well. Cytotoxicity was assessed microscopically after 3 days of incubation at 37°C. The toxin titre was defined as the reciprocal of the maximum dilution producing a cytopathic effect (rounding and detachment) on at least 50% of the cells in each well (CD₅₀/ml)².

### Proteomic analysis

Vero cell monolayers grown in 75 cm² flasks were treated for 3h with 1 µg/ml of either purified SubAB or the non-toxic mutant SubA_{A272}B. Treated Vero cells were solubilized, separated by 2D electrophoresis, and subjected to proteomic analysis as described in Supplementary Information. N-terminal amino acid sequence analysis was performed at the Australian Proteome Analysis Facility, Macquarie University, Sydney, Australia.

### Western blot analysis

Proteins were separated by SDS-PAGE and transferred onto nitrocellulose as described previously^{19,20}. Filters were probed with polyclonal goat anti-BiP (C-20, Santa Cruz Biotech, CA) (diluted 1:5000), followed by rabbit anti-goat IgG-alkaline phosphatase conjugate (BioRad Laboratories, CA). Labelled bands were visualized using NBT/X-phosphate substrate (Roche Molecular Diagnostics, Germany). The Benchmark™ Prestained Protein Ladder (Invitrogen, Carlsbad, CA) was used as a size marker.

### Confocal microscopy

Purified SubAB was labelled with Oregon Green (OG) using a Molecular Probes FluoReporter Oregon Green 488 Protein Labelling Kit (Invitrogen, Carlsbad, CA). This labelling had no discernible effect upon specific cytotoxicity for Vero cells. Vero cells grown on 1 cm coverslips in 24-well plates were treated with 1 µg/ml OG-SubAB in DMEM and after 60 min, were washed with PBS, and incubated for a further 30 min in fresh DMEM. Cells were then washed with PBS, fixed with 4% formaldehyde in PBS for 10 min and permeabilized with 0.1% Triton X-100. Coverslips were then washed in PBS, blocked with 20% FCS in PBS for 1 h at 37°C, and then treated with goat anti- BiP (C-20) (diluted in PBS/10% FCS) for 2 h at 37°C. After three washes with PBS, the coverslips were reacted with Texas Red- (TR)- conjugated rabbit anti-goat IgG (Invitrogen), diluted in PBS/10% FCS for 1 h at 37°C. Cells on the coverslips were then washed three times with PBS, twice with water, dried, and mounted on glass slides using 3 µl of ProLong Gold Antifade (Invitrogen), allowed to cure for 1-2 h, and then sealed with nail varnish. Cells were examined using a BioRad MRC 600 Dual-Fluorescence Confocal microscope. Images were captured using CoMOS software (BioRad) and image overlays were performed using Adobe Photoshop.

### DNA methods

Routine DNA manipulations were carried out essentially as described previously²¹. DNA sequencing employed dye-terminator chemistry and an ABI 3700 sequencer.

### Site-directed mutagenesis of BiP

A 2.0-kb fragment containing the murine BiP coding sequence was first excised from a full-length cDNA clone in the eukaryotic expression vector pCMV4²² (a gift from M.J. Gething, University of Melbourne) using *Xba*I, inserted into the prokaryotic expression vector pBC (Stratagene, La Jolla, CA) downstream of the vector *lac* promoter and ribosome binding site, and transformed into *E*. *coli* JM109²³. Expression of BiP by one of the resultant recombinant *E. coli* transformants was confirmed by Western blot analysis. Overlap extension PCR mutagenesis was then be used to construct a derivative encoding a BiP protein with a L₄₁₆→D substitution adjacent to the SubAB cleavage site. This involved two separate PCR amplifications of pBC-BiP DNA template, using the Expand™ High Fidelity PCR Kit (Roche Molecular Diagnostics, Germany). The first used the universal M13 Forward primer and primer L416DOLR (5'-ACAAACATCAA GGTCTACCAGATCACCTGTATCC-3') (SEQ ID NO:5). The second PCR used primer L416DOLF (5'-GGTGATCTGGTAGACCTTGATGTTTGTCCCCTTAC-3') (SEQ ID NO:6) and the universal M 13 Reverse primer. The two separate PCR products were then purified, combined, and the complete BiP coding region was reamplified using the M13 Forward and Reverse primers. The resultant PCR product was then cloned into pBC downstream of the vector *lac* promoter, and transformed into *E. coli* JM109. Recombinant plasmids were purified from the resultant transformants and subjected to sequence analysis to confirm that the correct mutation had been introduced. Expression of a BiP protein was also confirmed by Western blot analysis.

### Expression of BiP in transfected Vero cells

The native BiP and BiP_{D416} coding sequences were excised from pBC using *Xba*I, inserted into the eukaryotic expression vector pEFIRES-P ²⁴, and transformed into *E. coli* JM109. Purified plasmid DNA was extracted from transformants and subjected to restriction analysis to confirm correct orientation with respect to the vector promoter. Vero cells were then transfected with plasmid DNA using Lipofectamine 2000™ reagent as recommended by the supplier (Invitrogen). Transfected cells were selected using 5 µg/ml puromycin (Sigma).

### RESULTS

To identify the crucial cellular substrate(s) for the toxin, we conducted proteomic analysis of Vero cell monolayers that had been treated for 3 h with 1 µg/ml of either purified SubAB holotoxin or the inactive mutant SubA_{A272}B. 2D gel electrophoresis revealed only four spots specific to the sample treated with the active toxin. These were excised, digested with trypsin and identified by MALDI-MS analysis as 14-3-3 gamma, 14-3-3 epsilon, mitochondrial matrix protein p32, and BiP. Of these, only BiP (accession no. P11021), exhibited a significant size discrepancy (approx. 27 kDa on the gel compared with 72 kDa from the database) consistent with proteolytic cleavage by SubAB. Furthermore, the MALDI-MS analysis indicated that the tryptic peptides from the excised spot were derived exclusively from the C-terminal domain of BiP (residues 448-654).

BiP (Immunoglobulin Binding Protein) (also known as GRP78) is a highly conserved ER chaperone essential for survival of eukaryotic cells⁶⁻⁸. It comprises an N-terminal ATPase and a C-terminal protein-binding domain. One of its functions is to mediate correct folding of nascent secretory proteins, by binding exposed hydrophobic domains via the C-terminus, with subsequent release coupled to N-terminal domain-catalysed ATP-hydrolysis^{7,8}. BiP is also responsible for maintaining the permeability barrier of the ER membrane by sealing the lumenal end of the Sec61 translocon pore, as well as for targeting of terminally mis-folded proteins to the Sec61 apparatus for degradation by the proteasome^{8,9}. BiP plays a crucial role in the unfolded protein response as the ER stress-signalling master regulator⁷. It also exhibits anti-apoptotic properties through interference with caspase activation. Thus, disruption of BiP function has inevitably fatal consequences for the cell^{7,10-12}.

To confirm that SubAB mediates cleavage of BiP, Vero cells were incubated with 1 µg/ml purified SubAB or SubA_{A272}B for 3 h. Cell lysates were then subjected to SDS-PAGE and Western blot analysis using polyclonal goat anti-BiP (Fig. 1a). The BiP antibody labelled a 72-kDa species in the lysates of untreated Vero cells and those treated with the inactive toxin derivative. In contrast, the active SubAB-treated lysate had a markedly diminished 72-kDa band as well as a new antibody-reactive species at approximately 28 kDa. This is consistent with the size of the C-terminal BiP fragment detected by proteomic analysis (approx. 27 kDa). The fact that only a single cleavage product was detected is consistent with the fact that the antibody (C-20) was raised against a 20 amino acid peptide from the C-terminus of BiP. An additional approximately 68-kDa species reacted weakly with the antibody, but was not susceptible to cleavage by SubAB. However, this species did not react with a separate polyclonal BiP antibody (N-20, Santa Cruz Biotech), and so is presumed to be a cross-reacting protein. BiP cleavage was evident within 20 min of treatment with a dose of 1 µg/ml SubAB and was maximal within 60 min (Fig. 1b). There was also clear evidence of BiP cleavage in Vero cells after 1 h at doses of SubAB as low as 0.32 ng/ml (Fig. 1c). Cleavage of the 72-kDa BiP species was completely blocked when Vero cells were pre-incubated for 30 min with 0.5 µg/ml brefeldin A, which inhibits retrograde transport by disrupting the Golgi aparatus (Fig. 1d). Co-localization of SubAB with BiP in toxin-treated Vero cells could also be demonstrated by confocal microscopy using Oregon Green-labelled SubAB and C-20 anti-BiP (Fig. 2). Since BiP is located exclusively in the ER lumen⁸, co-localization with SubAB also confirms retrograde transport of the toxin to this compartment. BiP cleavage by SubAB was also demonstrated in human colonic epithelial (Hct8) and murine neuroblastoma (N2A) cell lines (result not shown, and Fig. 7a, respectively).

To confirm that BiP is a target for SubAB *in vivo,* mouse liver tissue was examined 24 h after intraperitoneal injection with 5 µg SubAB. SDS-PAGE and Western blot analysis with anti-BiP revealed significant cleavage of BiP in toxin-treated mice, but not in control mice or in those injected with 5 µg SubA_{A272}B (Fig. 3a). Injection of SubAB, but not SubA_{A272}B, also resulted in an unfolded protein response (UPR) in the liver at 24 h, as judged by induction of the transcription factor CHOP (Fig. 3b). CHOP induction was also evident in N2A cells after 24 h treatment with 1µg/ml SubAB or the known ER stressors tunicamycin or thapsigargin, but not after treatment with SubA_{A272}B or Shiga toxin (Fig. 7b).

Finally, to confirm that SubAB is directly responsible for cleavage of BiP, purified bovine BiP was tested *in vitro* as a substrate for purified SubAB or SubA_{A272}B, as judged by SDS-PAGE (Fig. 3c). Untreated BiP migrated as a single 72-kDa species, and was not affected by exposure to SubA_{A272}B. However, in the presence of active SubAB, BiP was cleaved into 44-kDa and 28-kDa fragments. This provides confirmation that SubAB directly cleaves BiP at a single position. Cleavage also occurred when BiP was treated with the purified catalytic subunit SubA, but it was not cleaved by purified SubB (Fig. 3c). The kinetics of BiP cleavage by SubAB vs SubA were indistinguishable (result not shown). The 28-kDa (presumptive C-terminal) BiP fragment was then excised from the gel and subjected to N-terminal sequence analysis. The first 10 residues had the sequence LDVCPLTLGI (SEQ ID NO:7), which matches amino acids 417-426 of BiP. This indicates that cleavage occurs between two leucine residues at positions 416 and 417 in the 654 amino acids BiP sequence; these are located in the exposed hinge that connects the ATPase and protein-binding domains¹³.

Most subtilisin-like enzymes are non-specific proteases that exhibit a preference to cleave after hydrophobic residues. However, our proteomic data suggested that SubAB targeted a single protein in mammalian cells. Moreover, SubAB did not cleave purified human Hsp70 and Hsc70 proteins *in vitro* (see Fig. 8a). These two members of the Hsp70 family are the closest homologues of BiP, and are identical at 7 of 11 amino acid positions flanking the cleavage site, including the di-leucine motif

Identification of the cleavage site provided an opportunity to definitively prove that the mechanism of cytotoxicity of SubAB is BiP cleavage. If a single amino acid substitution at this site rendered the modified BiP resistant to cleavage by the toxin, co-expression of such a protein in transfected Vero cells might protect them from SubAB toxicity. A full-length murine BiP cDNA was cloned into a prokaryotic expression vector (pBC) and mutated to encode a BiP derivative with a L₄₁₆→D substitution (see Methods). French pressure cell lysates of recombinant *E. coli* expressing either native or mutated BiP (designated BiP_{D416}) were treated with 1 µg/ml SubAB or SubA_{A272}B, and cleavage of BiP was assessed by Western blot analysis using C-20 anti-BiP. The native BiP clone lysate contained a 72-kDa immunoreactive species, which was susceptible to cleavage by SubAB, but not SubA_{A272}B. In contrast, the BiP_{D416} clone lysate contained a 72-kDa immunoreactive species, which was unaffected by either active or mutant toxin (Fig. 5a). Resistance of BiP_{D416} to cleavage was also confirmed by exposing purified protein to purified SubAB *in vitro* (see Supplementary Fig. 3b).

Next, Vero cells were transfected with BiP or BiP_{D416} coding sequences inserted into the eukaryotic expression vector pEFIRES-P (see Methods). Western blot analysis indicated almost complete digestion of BiP occurred when either Vero cells or those transfected with pEFIRES-P-BiP were exposed to 1 µg/ml SubAB for 2 h. However, in cells transfected with pEFIRES-P-BiP_{D416}, a proportion of the BiP remained intact after SubAB treatment, consistent with low-level co-expression of the resistant BiP_{D416} protein (Fig. 5b). These transfectants exhibited negligible cytotoxicity when exposed to 0.1 µg/ml SubAB for 3 days, whereas profound cytotoxicity was evident when untransfected Vero cells or those transfected with pEFIRES-P-BiP were exposed to the toxin (Fig. 5c). We also performed quantitative titrations of purified SubAB on Vero cells transfected with or without either pEFIRES-P-BiP or pEFIRES-P-BiP_{D416}. There was no difference in endpoint titre when SubAB was assayed on untransfected Vero cells or those transfected with the native BiP construct (200,000 CD₅₀/µg toxin). However, when assayed on Vero cells transfected with the BiP_{D416} construct, SubAB cytotoxicity was below the detectable threshold even at the maximum toxin concentration tested, and the "endpoint titre" was by definition < 50 CD₅₀/µg toxin. Confocal microscopy using Oregon Green-labelled SubAB indicated that there was no defect in uptake or intracellular trafficking of the toxin in the transfected cells (result not shown). Thus, resistance to SubAB cytotoxicity is a direct consequence of co-expression of BiP_{D416}.

The findings of this study indicate that SubAB is unique in two ways. Unlike other bacterial AB₅ cytotoxins, it does not need to be translocated from the ER lumen into the cytosol in order to interact with its cellular target. For Ctx and Stx, this retro-translocation is achieved through subversion of the Sec61 translocon apparatus, which is normally used to transport terminally misfolded host proteins from the ER lumen into the cytosol ready for degradation, a process in which BiP is also involved^{15,16}. Most importantly, however, no other cytotoxin has been shown to directly target chaperone proteins or components of the ER.

### Proteomic analysis.

Vero cell monolayers grown in 75 cm² flasks were treated for 3h with 1 µg/ml of either purified SubAB or the non-toxic mutant SubA_{A272}B. Treated Vero cells were solubilized and subjected to 2D electrophoresis according to the method of O'Farrell²⁵ by Kendrick Laboratories (Madison, WI). Isoelectric focusing was carried out in glass tubes of inner diameter 3.5 mm, using 2% pH 3.5-10 ampholines (LKB/Pharmacia, Baltimore, MD) for 20,000 volt-h. 50 ng of an IEF internal standard, tropomyosin, was added to each sample prior to loading. After equilibrium in SDS sample buffer (10% glycerol, 50 mM dithiothreitol, 2.3% SDS and 0.0625 M Tris, pH 6.8), each tube gel was sealed to the top of a stacking gel that overlays a 10% acrylamide slab gel (0.75 mm thick). SDS slab gel electrophoresis was carried out for about 5 h at 25 mA/gel. The following proteins (Sigma Chemical Co, St. Louis, MO) were added as molecular weight standards to the agarose sealing the tube gel to the slab gel: myosin (220,000), phosphorylase A (94,000), catalase (60,000), actin (43,000) carbonic anhydrase (29,000) and lysozyme (14,000). 2D gels of Vero cells treated with SubAB or SubA_{A272}B are shown in Fig. 6.

Duplicate stained 2D gels of both active and inactive toxin-treated samples were then scanned with a laser densitometer (Model PDSI; Molecular Dynamics, Sunnyvale, CA) and the images were analyzed using Progenesis Discovery software (version 2005; Nonlinear Technology). All major spots and all changing spots were outlined, quantified and matched on all the gels. The general method of computerized analysis for these pairs included automatic spot finding and quantification, automatic background subtraction (Progenesis mathematical model) and automatic spot matching in conjunction with detailed manual checking of the spot finding and matching functions. This analysis resolved >900 discrete protein spots, of which four were unique to the SubAB-treated sample. These were excised, digested with trypsin and subjected to MALDI-MS analysis at the Protein Chemistry Core Facility, Columbia University, New York; spots were identified by comparison of the peptide mass fingerprints with protein sequence databases (Table 1).

**Table 1. Protein Identification**

| Spot # | Protein identified | Accession # | pI from gel (approx.) | MW from gel (approx.) | MW from database |
|---|---|---|---|---|---|
| 620 | 14-3-3 epsilon | 55741616 | 5.13 | 30,404 | 29,141 |
| 625 | Mitochondrial matrix protein p32 | 8248258 | 4.96 | 28,711 | 31,407 |
| 639 | 14-3-3 gamma | P61981 | 5.09 | 30,122 | 28,271 |
| 666 | BiP/GRP78 | P11021 | 5.38 | 26,995 | 72,251 |

### EXAMPLE 2 - CONSTRUCTION OF DNA SEQUENCES ENCODING EGF-SUBA FUSION PROTEIN

### GENERAL DESCRIPTIONS

### Bacterial Strains, Plasmids, and Mammalian Cells

*E. coli* strains NovaBlue and BL21(DE3) are commercially available from Novagen (USA). Vector pET/C4(G₄S)₃ for bacterial expression of recombinant proteins with an N-terminal Cys-tag was made in SibTech, Inc³². Parental rat glioma cells (F98) and their derivatives F98/EGFR engineered to express 10⁶ EGFR per cell were kindly provided by Dr. R. Barth (The Ohio State University, Columbus, OH, USA). MDA-231luc, a luciferase-expressing derivative of MDA-MB-231 human breast carcinoma cells (ATCC # HTB-26™) were developed in Sibtech, Inc. as described earlier²⁹. EGFR expression level in MDA231 luc was estimated by flow-cytometry using EGFR-specific antibody (AbCam, Inc.USA) with F98/EGFR cells serving as a standard. All cells were maintained in DMEM supplemented with 10% fetal bovine serum (Gemini, Inc., USA), 2 mM L-glutamine and antibiotics at 37°C, 5% CO₂.

### DNA Manipulations

The restriction and modification enzymes employed herein are commercially available from the usual sources and were used according to manufacturer's instructions. The sequencing of the different DNA constructs was done at Macromolecular Resources (Department of Biochemistry and Molecular Biology, Ft. Collins, CO, USA). Competent cells, transformation, and bacterial media were prepared according to Sambrook et al. (J. Sambrook, E. F. Fritsch and T. Maniatis. (1989) Molecular Cloning: A Laboratory Manual, Cold Spring Harbor Laboratory Press, Cold Spring Harbor, NY), or according to the manufacturer's instructions. Purification of plasmids was done using Mobuis DNA Purification System (Novagen, USA) according to the manufacturer's instructions. Further purification of DNA as well as purification of DNA from agarose gels was done using the GeneClean Spin kit (Bio 101, USA) according to the manufacturer's instructions.

### SUB-CLONING OF 22-347 AMINO ACID FRAGMENT OF SUBAB SUBUNIT A AND HUMAN EGF INTO THE PET/C4(G₄S)₃ EXPRESSION VECTOR.

### Primers for amplification of DNA encoding human EGF and 22-347aa fragment of SubAB Subunit A

All primers were synthesized by GeneLink (NY, USA). The primers corresponding to the "sense" (SEQ ID NO:1) and "antisense" (SEQ ID NO:2) strands of human EGF included NcoI restriction sites to both termini. To compensate OFR shift due to cloning into NcoI, di-nucleotide GA (shown in bold) was inserted in SEQ ID NO:1, and one nucleotide A (bold) was inserted in SEQ ID NO:2
5'- CACAAGCCATGG**GA**AACAGCGATAGCGAATGCCCG -3' (SEQ ID NO:1)
5'- ACTACCCATGG**A**GCGCAGTTCCCACCATTTCAG-3' (SEQ ID NO:2)

The primer corresponding to the "sense" strand of a 22-347aa fragment of SubAB subunit A (SEQ ID NO:3) introduced BamHI site immediately upstream of a codon for Glu22. The "antisense" (SEQ ID NO:4) primer introduced a stop-codon immediately downstream of a codon for Leu347 followed by BamHI restriction site.
5'- CACGGATCCGAAAAACCCTGGTATTTTGATGC -3' (SEQ ID NO:3)
5'- CACGGATCC TTACAG TTCTTC ACTC TTCCC -3' (SEQ ID NO:4)

Two DNA fragments encoding full-length EGF and a 22-347aa fragment of SubAB subunit A followed by a stop-codon were amplified by PCR using a high fidelity TGO polymerase (Roche Diagnostics, Germany) according to manufacturer's instructions. DNA fragment encoding EGF and pET/C4(G₄S)₃ plasmid DNA were digested with NcoI, ligated together and transformed into NovaBlue bacteria. Clones with correct orientation of EGF ORF were selected by colony PCR using a T7-promoter based sense primer and SEQ ID NO:2. Plasmid DNA isolated from one selected clone was designated pET/C4(G₄S)₃EGF and confirmed by sequencing.

PCR amplified DNA encoding a 22-347aa fragment of SubAB subunit A with a stop-codon and pET/C4(G₄S)₃EGF plasmid DNA were digested with BamHI, purified from agarose gel, ligated and transformed into NovaBlue host. Clones with correct orientation of SubA were selected by colony PCR using SEQ ID NO:1 and SEQ ID NO:4 primers. Plasmid DNA isolated from one selected clone was designated pET/C4(G₄S)₃EGF-SubA and confirmed by sequencing.

The pET/C4(G₄S)₃EGF-SubA plasmid DNA encodes EGF-SubA fusion protein (SEQ ID NO:13) comprising an initial Met, a 15 amino acid Cys-tag (SEQ ID NO:10), a 17 amino acid linker (SEQ ID NO:11), 53 amino acid human EGF (SEQ ID NO:9, a 7 amino acid linker (SEQ ID NO:12), and a 326 amino acid SubA fragment (SEQ ID NO:8) (Fig. 8).
SEQ ID NO:10
   Met Lys Glu Ser Cys Ala Lys Lys Phe Gln Arg Gln His Met Asp Ser
SEQ ID NO:11
   Gly Gly Gly Gly Ser Gly Gly Gly Gly Ser Gly Gly Gly Gly Ser Met Gly
SEQ ID NO:12
   Ser Met Ala Asp Ile Gly Ser

### EXAMPLE 3 - EXPRESSION AND PURIFICATION OF RECOMBINANT EGF-SubA.

The pET/C4(G₄S)₃EGF-SubA transformed *E. coli* cells BL21(DE3) were grown in LB broth containing 34 mg/L kanamycin at 37°C and 300 rpm shaking to the middle of the log phase of growth as detected by optical density at 600 nm. IPTG (isopropyl-β-D-thiogalactoside, Life Technologies, USA) was added to the culture to a final concentration of 1 mM to induce protein expression. Induced cultures were grown for 2 hours harvested by centrifugation (25 min, 5000 x g) and ruptured using high-pressure homogenizer (Avestin, Canada) according to manufacturer's instructions. Soluble and insoluble parts of bacterial homogenate were separated by centrifugation (20 min, 10,000 x g at 4°C). Reducing SDS-PAGE analysis indicated that EGF-SubA fusion protein was present exclusively in insoluble part (Fig. 9). The inclusion body pellets were washed with the buffer containing 20 mM Tris-HCL, pH 8.0, 0.5 M NaCl and solubilized in 8 M urea supplemented with 20 mM Tris-HCl pH 8.0, 150 mM NaCl. Solubilized protein was reduced by treatment with 10 mM DTT for 20 hrs at 4°C, followed by treatment with 2.5 mM TCEP for 24 h at 4°C, and then refolded via a 3-step dialysis. Step 1, in a 20-fold volume of 4 M urea, 0.5 M arginine, 1 mM reduced glutathione, 0.4 mM oxidized glutathione, 20 mM Tris-HCl pH 8.0. Step 2, in 20 volumes of 0.5 M urea, 0.5 M arginine, 20 mM Tris-HCl pH 8.0. Step 3, in 100 volumes of 0.01% sodium deoxycholate, 20 mM Tris-HCl pH 9.0. Refolded EGF-SubA fusion protein was purified by ion-exchange chromatography on HiTrap Q Fast Flow Sepharose (1-ml pre-packed columns, GE Healthcare, USA) to purity of greater than 95% (Fig. 9) and stored in small aliquots at -20°C. The concentration of EGF-SubA was determined by the integral absorption at 216 nm on RP-HPLC profiles.

### EXAMPLE 4 - FUNCTIONAL ACTIVITY OF EGF AND SUBA MOIETIES IN EGF-SUBA FUSION PROTEIN

### A. Induction of EGFR tyrosine phosphorylation by EGF moiety, of EGF SubA

To evaluate functional activities of EGF and SubA moieties in the fusion protein we used F98/EGFR rat glioma cells, engineered to express 10⁶ EGFR per cell. For induction of EGFR tyrosine phosphorylation, cells were plated onto 24-well plates 80,000 cell/well, incubated for 6 hrs at 37°C, 5% CO₂ and shifted to serum-free medium for 16-hour incubation at 37°C, 5% CO₂. Subsequently, the medium was changed to serum-free DMEM supplemented with 0.1 mM sodium orthovanadate, 100 ng/ml bovine serum albumin, 25 mM HEPES pH 7.2, and cells were incubated for 10 min at 37°C. Cells were stimulated with varying amounts of EGF-SubA or control EGF (Sigma) for 10 min at 37°C, lysed, fractionated by SDS-PAGE on 7.5% gels and transferred to nitrocellulose (BioRad Laboratories, USA) for probing with anti-phosphotyrosine antibody (clone PT-66, Sigma) according to the manufacturer's instructions. Figure 10A demonstrates that EGF-SubA and control EGF induced EGFR tyrosine autophosphorylation in the same concentration range, indicating that fusion of N-terminal Cys-tag and C-terminal SLiP to EGF did not affect significantly its ability to bind and activate EGFR.

### B. Cleavage of GRP78/BiP by SubA moiety of EGF-SubA

MDA231 luc, F98/EGFR and parental F98 cells were plated onto 6-well plates, 500,000 cell/well, in complete culture medium. Twenty hours later, EGF-SubA was added to triplicate wells to a final concentration of 1 nM. Cells were incubated at 37°C, 5% CO₂ for 6, 12 and 24 hrs, then collected, lyzed, fractionated by SDS-PAGE on 15% gels and transferred to nitrocellulose for probing with GRP78/BiP-specific antibody (Santa Cruz, USA) and control β-□actin-specific antibody (Sigma) according to the manufacturer's instructions. Horseradish peroxidase conjugated anti-rabbit IgG (GE Healthcare, USA) was applied as recommended by manufacturer, and visualized by ECL Plus kit (GE Healthcare). The appearance of a 28-kDa fragment GRP78/BiP and enhanced expression of intact protein migrating as a 72-kDa band was detected after a 6-h exposure to 1 nM EGF-SubA, indicating that EGF-SubA retained the substrate specificity of parental SubAB toxin.

### C. Selective cytotoxicity of EGF SubA to EGFR-positive cancer cells

MDA231luc, F98/EGFR and parental F98 cells were plated onto 96-well plates, 2,000 cell/well, in complete culture medium. Varying amounts of EGF-SubA were added to duplicate wells 20 hrs later. For competition assay, EGF was titrated in complete culture medium supplemented with EGF-SubA and added to duplicate wells with MDA231luc cells to a final concentration of 50 pM EGF-SubA and EGF ranging from 10 pM to 1 nM. Plates were incubated for 72 hours at 37°C, 5% CO₂ and cells were quantitated using a Cell Proliferation Assay kit (Promega, USA).

EGF-SubA was 500-fold more cytotoxic toward F98/EGFR (IC₅₀ of 5 pM, Fig. 11A) than to parental F-98 cells (IC₅₀ of ∼2.5 nM). High toxicity of EGF-SLiP to F98/EGFR cells provided an evidence of SubA functional activity as a part of a fusion protein, whereas 500-fold difference in IC₅₀ values for EGFR-overexpressing *vs* EGFR-negative cells confirmed EGFR-mediated molecular targeting.

EGF-SubA was highly toxic for MDA-231luc cells with an IC₅₀ of ∼50 pM (Fig. 11B), and this toxicity was inhibited by excess of EGF (Fig. 11C), confirming its EGFR-dependent mechanism. MDA-231luc cells appeared to be 10-fold less sensitive to EGF-SLiP than engineered F98/EGFR cells (IC₅₀ of 5 pM), reflecting, most probably, the difference in EGFR expression levels readily detectable by flow cytometry (data not shown).

### EXAMPLE 5 - ENHANCED CYTOTOXICITY OF THAPSIGARGIN AND VELCADE IN THE PRESENCE OF EGF-SUBA FUSION PROTEIN.

MDA231luc and F98/EGFR cells were plated on 96-well plates and quantitated after 72 hours of drug exposure as described in EXAMPLE 3B. Twenty hours after plating, thapsigargin and velcade were added to duplicate wells, either alone or in combination with EGF-SubA. For combination drug/EGF-SubA treatments, the following molar drug-to-protein ratios were used: thapsigargin:EGF-SubA ratio of 1000:1 (Fig. 12A); Velcade:EGF-SubA ratio of 200:1 (Fig. 12B).

### EXAMPLE 6 - ENHANCED THAPSIGARGIN-INDUCED APOPTOSIS IN EGFR-POSITIVE CELLS IN THE PRESENCE OF EGF-SUBA

MDA2311uc were plated on 6-well plates, 500,000 cells/well. Twenty hours later, drugs were added to triplicate wells to the following final concentrations: thapsigargin alone, 1 µM; EGF-SubA alone, 1 nM, or their mixture to the same final concentrations. Cells were incubated for 24 hrs under normal culture conditions, then harvested in PBS supplemented with 2 mM EDTA (Invitrogen, USA), spun down at 4,000 x g for 5 min and lysed at 4 °C for 5 min in a lysis buffer (250 mM sucrose, 20 mM HEPES pH 7.4, 1% NP-40, 10 mM KCl, 1 mM EDTA, 25 µg/ml antitripsin, 25 µg/ml aprotenin). Cell lysates were clarified by centrifugation (10,000 x g for 2 min) and supplemented with 2 mM DTT to preserve caspase enzymatic activity. Total protein in cytosolic fractions was determined by Bio-Rad protein assay (Bio-Rad, Hercules, CA). Aliquots containing 40 µg of protein were analyzed in a final volume of 100 µl of caspase activity buffer (0.1 M HEPES pH 7.4, 2 mM DTT, 0.1% NP-40, 1% sucrose) containing 50 µM of the following caspase substrates: pan-caspase substrate AC-2 (DEVD-AFC, Calbiochem, USA) and caspase-3/7-specific substrate (Whole Cell Apoptosis Assay kit, Beckman Coulter, USA). Reaction mixtures were incubated for 1 hour at 37 °C, and fluorescence was measured at λₑₓ = 400 nm and λ em = 505 nm.

To test functional activity of EGF-SubA under conditions of thapsigargin-induced enhanced expression ofGRP78/BiP, cell lysates containing 40 µg of protein were analyzed by Western blotting with GRP78/BiP- specific antibody (Fig. 13B). Western blot analysis of the same lysates (40 µg of protein, each) was also done with α-fodrin-specific antibody (Chemicon, USA) to confirm enhanced apoptosis, which means greater accumulation of 120-kDa and 150-kDa fragments, in thapsigargin/EGF-SubA combination treatment regimen (Fig. 13C).

### EXAMPLE 7 - ENHANCED SENSITIVITY OF DOXORUBICIN-RESISTANT CELLS TO EGF-SUBA.

Doxorubicin-resistant MDA231luc-Dox and F98/EGFR-Dox cells were selected by maintaining corresponding parental cells, MDA231luc and F98/EGFR, in the presence of 20 nM and 200 nM doxorubicin, respectively, for 8-10 weeks in 75-cm flasks. Effects of EGF-SubA on MDA231luc, MDA231luc-Dox, F98/EGFR, and F98/EGFR-Dox were determined in simultaneous experiments under the same conditions as described in Example 3C. Enhanced sensitivity of doxorubicin-resistant cells to EGF-SubA relative to parental cells F98/EGFR (Fig. 14a) and MDA231luc (Fig. 14b) was readily detectable resulting in significant decrease in the numbers of viable doxorubicin-resistant cells at the EGF-SubA concentration range that was non-toxic or marginally toxic for the corresponding parental cells and in a significant decrease in IC50 values for both cell types.

### EXAMPLE 7 DEVELOPING VEGF-TARGETED SUBAB SUBUNIT A.

SUB-CLONING OF a 22-347 amino acid fragment of SubAB subunit A and a 3-112 fragment of human VEGF into the pET/C4-G4S EXPRESSION VECTOR.

### Primers for amplification of DNA encoding a fragment of human VEGF

PCR amplification of VEGF was done as described in Example 1. The primers corresponding to the "sense" (SEQ ID NO:5) and "antisense" (SEQ ID NO:6) strands of human VEGF included NcoI restriction sites to both termini. To compensate OFR shift due to cloning into NcoI, one nucleotide C (bold, underlined) was inserted in SEQ ID NO:14
5'- ACTACCCATGG**C**TCTTGCTCTATCTTTCTTTGGTCTGC-3' (SEQ ID NO:14)
5'- CACAAGCCATGGCACCCATGGCAGAAGGAGGA-3' (SEQ ID NO:15)

DNA fragment encoding 3-112 amino acid long VEGF fragment and pET/C4-G4S plasmid DNA were digested with NcoI, ligated together and transformed into NovaBlue bacteria. Clones with correct orientation of EGF ORF were selected by colony PCR using a T7-promoter based sense primer and SEQ ID NO:6. Plasmid DNA isolated from one selected clone was designated pET/C4-G4S-VEGF110NoStop and confirmed by sequencing.

PCR amplified DNA encoding a 22-347aa fragment of SubAB subunit A with a stop-codon, digestion with BamHI, and ligation into BamHI-digested pET/C4-G4S-VEGF110NoStop was done as described in Example 1. Clones with correct orientation of SubA were selected by colony PCR using SEQ ID NO:14 and NO:15 primers. Plasmid DNA isolated from one selected clone was designated pET/C4-G4S-VEGF110-SubA and confirmed by sequencing.

The pET/C4-G4S-VEGF 110-SubA plasmid DNA encodes VEGF-SubA fusion protein (SEQ ID NO:17), comprising an initial Met, a 15 amino acid Cys-tag (SEQ ID NO:10), a 5 amino acid linker (SEQ ID NO:18), 110 amino acid fragment of human VEGF₁₂₁ (SEQ ID NO:16), a 7 amino acid linker (SEQ ID NO:12), and a 326 amino acid SubA fragment (SEQ ID NO:8), (Fig. 15a). SEQ ID NO:18
Gly Gly Gly Gly Ser

### Bacterial expression of VEGF-SubA

Expression ofVEGF-SubA in E. coli strain Origami2(DE3) (Novagen), refolding from inclusion bodies and purification by ion-exchange chromatography was done as described for EGF-SubA fusion protein.

SUB-CLONING OF a 22-347 amino acid fragment of SubAB subunit A into the pET/C4-G4S EXPRESSION VECTOR To Obtain Cys-tagged SubA protein for site-specific conjugation to Cys-tagged VEGF and other proteins.

### Cloning and bacterial expression of Cys-SubA.

PCR amplified DNA encoding a 22-347aa fragment of SubAB subunit A with a stop-codon (described in Example 1) and pET/C4-G4S plasmid DNA (SibTech, Inc.) were digested with BamHI, purified from agarose gel, ligated and transformed into NovaBlue host as described in Example 1. Clones with correct orientation of SubA were selected by colony PCR using a T7-based sense primer and SEQ ID NO:4 antisense primer. Plasmid DNA isolated from one selected clone was designated pET/C4-G4S-SubA and confirmed by sequencing.

The pET/C4-G4S-SubA plasmid DNA encodes Cys-SubA fusion protein (SEQ ID NO:19) comprising an initial Met, a 15 amino acid Cys-tag (SEQ ID NO:10), a 9 amino acid linker (SEQ ID NO:20), and a 326 amino acid SubA fragment (Fig. 15b) (SEQ ID NO:8). SEQ ID NO:20
Gly Gly Gly Gly Ser Met Ala Asp Ile Gly Ser

Bacterial expression of Cys-SubA in E. coli strain BL21(DE3) and refolding from inclusion bodies was done as described for EGF-SubA fusion protein. SP column chromatography (1-ml pre-packed columns from GE Healthcare) was used for final purification of Cys-SubA.

For site-specific conjugation of Cys-SubA to targeting proteins, C4-thiol group in Cys-tag is released from mixed disulfide bond with glutathione, as earlier described²⁷ and is used for conjugation using bi-functional cross-linkers that react with C4-thiol group and with an appropriate group in a targeting protein. In another approach, that may be suitable for proteins with the accessible thiol group that include but not limited to Cys-tagged proteins, disulfide bond might be established directly between said thiol group and C4 thiol group in Cys-SubA. In a specific example C4-thiol group in Cys-tagged scVEGF (SEQ ID NO: 21) is released from mixed disulfide bond with glutathione as described²⁷, activated with dithiodipyridine (DDP, Sigma) and then crosslinked to C4-thiol group in Cys-tag of Cys-SubA, resulting in SubA-S-S-scVEGF conjugate (Fig. 15b).

### REFERENCES

1. Fan et al., (2000) Curr. Opin. Struct. Biol. 10, 680-686.
2. Paton et al., (2004) J. Exp. Med. 200, 35-46.
3. Paton and Paton (1998) Clin. Microbiol. Rev. 11,450-479.
4. Paton and Paton (2005). J. Clin. Microbiol. 43, 2944-2947.
5. Siezen and Leunissen, (1997) Protein Science 6, 501-523.
6. Kim and Arvan, (1998). Endocrine Rev. 19, 173-202.
7. Hendershot (2004) Mt. Sinai J. Med. 71, 289-297.
8. Gething (1999) Cell Devel. Biol. 10, 465-472.
9. Hamman et al., (1998) Cell 92, 747-758**.**
10. Lee (2005) Methods 35, 373-381.
11. Rao et al., (2004) Cell Death Differ. 11, 372-380.
12. Rao et al. (2002) FEBS Lett. 514, 122-128.
13. Jiang et al., (2005) Mol. Cell. 20, 513-524.
14. Le Bonniec et al., (1992) J. Biol. Chem. 267, 19341-19348.
15. Lencer and Tsai (2003) Trends Biochem. Sci. 28, 639-645.
16. Yu and Haslam et al., (2005). Infect. Immun. 73, 2524-2532.
17. Macario and Conway de Macario (2005) N. Engl. J. Med. 353, 1489-1501.
18. Talbot et al., (2005) Infect. Immun. 73, 4432-4436.
19. Laemmli (1970) Nature 227, 680-685.
20. Towbin et al., (1979) Proc. Natl. Acad. Sci. USA. 76, 4350-4354.
21. Maniatis et al., (1982) Molecular cloning: a laboratory manual. Cold Spring Harbor Laboratory, Cold Spring Harbor, New York.
22. Kozutsumi et al. (1989). J. Cell. Sci. Suppl. 11, 115-137.
23. Yanisch-Perron et al., (1985) Gene 33, 103-119.
24. Hobbs et al., (1998) Biochem. Biophys. Res. Commun. 252, 368-372.
25. O'Farrell J. Biol. Chem. 250, 4007-4021 (1975).
26. Backer et al., (2005) Mol. Cancer Ther. 4, 1423-9.
27. Backer et al., (2006a) Biomaterials, 27:5452-8.
28. Backer et al., (2006b) Bioconjugate Chem. 17: 912-9
29. Backer et al., (2007a) Nature Med, 13: 504-509
30. Backer et al., (2007b) Antimicrobial Agents & Chemotherapy 51(1): 245-51, 2007b.
31. Backer and Backer. Bioconjugate Chem 12:1066-73, 2001.
32. Backer et al., (2007c). in Methods in Molecular Medicine, The Humana Press Inc. (in press).
33. Blankenberg et al., (2006). Eur. J. Nucl. Med. Mol. Imag. 33, 841-848.
34. Carew et al., (2006) Blood. 107(1):222-31.
35. Corazzari et al., (2007) Brit.J. Cancer. 96(7):1062-71.
36. Dong et al., (2005) Cancer Res. 65(13):5785-91.
37. Kim et al., (2006) Apoptosis. 11(1):5-13.
38. Li and Lee (2006) Curr. Mol. Med. 6(1):45-54**.**
39. Mese et a/.,(2001) Anticancer Res. 21(2A):1029-33
40. Pyrko et al., (2007) Mol. Cancer Ther. 6(4):1262-75.
41. Ranganathan et al., (2006) Cancer Biol. Ther. 5(7):729-35.
42. Yoshida (2007) FEBS J. 274(3):630-58..
43. Li et al., Journal of Cellular Physiology 153; 575-582

## Claims

1. The use of SubA in the manufacture of a medicament for the treatment of tumors.

2. The use according to claim 1 wherein SubA is provided combined with SubB (subunit B of subtilase cytotoxin) as a holotoxin.

3. The use according to any of claims 1 or 2, wherein SubA is provided as an active variant of SubA, the active variant being a fusion protein or a protein conjugate comprising SubA or a fragment thereof and further comprising a receptor or an antigen binding moiety, said receptor or antigen binding moiety being capable of binding a receptor or an antigen exposed on the surface of a target cell.

4. The use according to claim 3, wherein the target cell is a tumor cell.

5. The use according to claim 3, wherein the target cell is a vascular endothelial cell.

6. The use according to claim 3, wherein the receptor binding moiety is vascular endothelial growth factor.

7. The use according to claim 3, wherein the receptor binding moiety is epidermal growth factor.

8. The use according to any of the preceding claims, wherein the medicament further comprises an ER stress inducer, the ER stress inducer being selected from the group consisting of inhibitors of calcium dependent endoplasmic reticulum ATPase, inhibitors of proteasome, inhibitors of protein glycosylation, and compounds capable of inducing glucose or oxygen deprivation by targeting tumor angiogenesis and tumor vasculature.

## Patentansprüche

1. Verwendung von SubA bei der Herstellung eines Medikaments zur Behandlung von Tumoren.

2. Verwendung gemäß Anspruch 1, wobei SubA in Kombination mit SubB (Untereinheit B des Subtilase-Cytotoxins) als Holotoxin bereitgestellt wird.

3. Verwendung gemäß einem der Ansprüche 1 oder 2, wobei SubA als aktive Variante von SubA bereitgestellt wird, wobei die aktive Variante ein Fusionsprotein oder ein Proteinkonjugat ist, das SubA oder ein Fragment davon umfasst und weiterhin eine rezeptor- oder antigenbindende Struktureinheit umfasst, wobei die rezeptor- oder antigenbindende Struktureinheit einen Rezeptor oder ein Antigen, die auf der Oberfläche einer Zielzelle exponiert sind, binden kann.

4. Verwendung gemäß Anspruch 3, wobei die Zielzelle eine Tumorzelle ist.

5. Verwendung gemäß Anspruch 3, wobei die Zielzelle eine Gefäßendothel-Zelle ist.

6. Verwendung gemäß Anspruch 3, wobei die rezeptorbindende Struktureinheit ein Gefäßendothel-Wachstumsfaktor ist.

7. Verwendung gemäß Anspruch 3, wobei die rezeptorbindende Struktureinheit ein Epidermis-Wachstumsfaktor ist.

8. Verwendung gemäß einem der vorstehenden Ansprüche, wobei das Medikament weiterhin einen ER-Stressinduktor umfasst, wobei der ER-Stressinduktor aus der Gruppe ausgewählt ist, die aus Inhibitoren der calciumabhängigen ER-ATPase, Inhibitoren des Proteasoms, Inhibitoren der Proteinglycosylierung und Verbindungen, die Glucose- oder Sauerstoffentzug induzieren können, indem sie an der Tumorangiogenese und Tumorvaskulatur angreifen, besteht.

## Revendications

1. Utilisation de SubA dans la fabrication d'un médicament pour le traitement de tumeurs.

2. Utilisation selon la revendication 1, dans laquelle SubA est fournie combinée avec SubB (sous unité B de cytotoxine subtilase) comme holotoxine.

3. Utilisation selon l'une quelconque des revendications 1 ou 2, dans laquelle SubA est fournie en tant que variante active de SubA, la variante active étant une protéine de fusion ou un conjugué de protéine comprenant SubA ou un fragment de celle-ci et comprenant en outre un fragment de liaison à un récepteur ou antigène, le fragment de liaison à un récepteur ou antigène étant capable de se lier à un récepteur ou antigène exposé à la surface d'une cellule cible.

4. Utilisation selon la revendication 3, dans laquelle la cellule cible est une cellule tumorale.

5. Utilisation selon la revendication 3, dans laquelle la cellule cible est une cellule endothéliale vasculaire.

6. Utilisation selon la revendication 3, dans laquelle le fragment de liaison à un récepteur est un facteur de croissance endothéliale vasculaire.

7. Utilisation selon la revendication 3, dans laquelle le fragment de liaison à un récepteur est un facteur de croissance épidermique.

8. Utilisation selon l'une quelconque des revendications précédentes, dans laquelle le médicament comprend en outre un stimulus de stress ER, le stimulus de stress ER étant sélectionné parmi le groupe constitué d'inhibiteurs de ATPase de réticulum endoplasmique fonction du calcium, des inhibiteurs de protéasome, des inhibiteurs de glycosylation de protéine, et des composés capables d'induire une privation de glucose ou d'oxygène en ciblant l'angiogénèse tumorale et une vasculature tumorale.
